# EUROPEAN PATENT APPLICATION

(11) **EP 3 995 573 A1**
(43) Date of publication of application: **11.05.2022**
(21) Application number: 20836321.8
(22) Date of filing: 03.07.2020
(51) Int. Cl.: C12N 7/01, C07K 14/11, C12N 15/44, C12N 15/63, A61K 39/145, A61K 35/76, A61P 31/12, A61P 31/16, C12R 1/93

(54) **PROTEOLYTIC TARGETED VIRUS, LIVE VACCINE THEREOF, PREPARATION METHOD THEREFOR AND USE THEREOF**

(30) Priority: 05.07.2019 CN 201910603698
(71) Applicant: Si, Longlong, Shenzhen (CN); Zhen, Zhaoyu, Beijing 100085 (CN); Niu, Siwen, Beijing, 100085 (CN)
(72) Inventor: Si, Longlong, Shenzhen (CN); Zhen, Zhaoyu, Beijing 100085 (CN); Niu, Siwen, Beijing, 100085 (CN)
(74) Representative: Potter Clarkson
(86) International application number: PCT/CN2020/100086
(87) International publication number: WO 2021/004389

(57) **Abstract**

Provided is a proteolysis-targeting virus, wherein one or more proteolysis-targeting molecules that can be recognized by the ubiquitin-proteasome system are comprised at one or more different sites of protein thereof, and the viral protein is linked to the proteolysis-targeting molecules by one or more linkers that can be selectively cleaved. Also provided are a nucleic acid molecule encoding the proteolysis-targeting virus, a nucleic acid vector expressing the proteolysis-targeting virus, a preparation method for the proteolysis-targeting virus, methods for the preparation of an attenuated live virus, replication-incompetent live virus, replication-controllable live virus, and a relevant vaccine and medication for preventing and treating virus infections, a vaccine or pharmaceutical composition comprising the proteolysis-targeting virus, and a system for preparing the proteolysis-targeting virus.

Sequence listing

<110>  SI,Longlong
       ZHEN,Zhaoyu
       NIU,Siwen

<120>  Proteolysis-Targeting Virus, Live Vaccine thereof, Preparation Method
and Use thereof

<130>  EIC20310019P

<150>  2019106036989
<151>  2019-07-05

<160>  170

<170>  SIPOSequenceListing 1.0

<210>  1
<211>  7
<212>  PRT
<213>  Artificial Sequence

<400>  1
Ala Leu Ala Pro Tyr Ile Pro
1               5

<210>  2
<211>  10
<212>  PRT
<213>  Artificial Sequence

<400>  2
Asp Arg His Asp Ser Gly Leu Asp Ser Met
1               5                   10

<210>  3
<211>  40
<212>  PRT
<213>  Artificial Sequence

<400>  3
Ser His Gly Phe Pro Pro Glu Val Glu Glu Gln Asp Asp Gly Thr Leu
1               5                   10                  15
Pro Met Ser Cys Ala Gln Glu Ser Gly Met Asp Arg His Pro Ala Ala
            20                  25                  30
Cys Ala Ser Ala Arg Ile Asn Val
        35                  40

<210>  4
<211>  30
<212>  PRT
<213>  Artificial Sequence

<400>  4
Phe Val Asn Gln His Leu Cys Gly Ser His Leu Val Glu Ala Leu Tyr
1               5                   10                  15
Leu Val Cys Gly Glu Arg Gly Phe Phe Tyr Thr Pro Lys Ala
            20                  25                  30

<210>  5
<211>  6
<212>  PRT

<213>  Artificial Sequence

<220>
<221>  SITE
<222>  (4)..(4)
<223>  XAA indicates any amino acid


<220>
<221>  SITE
<222>  (5)..(5)
<223>  XAA indicates any amino acid


<400>  5
Asp Ser Gly Xaa Xaa Ser
1               5

<210>  6
<211>  10
<212>  PRT
<213>  Artificial Sequence

<220>
<221>  SITE
<222>  (7)..(7)
<223>  XAA indicates any amino acid


<220>
<221>  SITE
<222>  (8)..(8)
<223>  XAA indicates any amino acid



<400>  6
Asp Arg His Asp Ser Gly Xaa Xaa Ser Met
1               5                   10

<210>  7
<211>  12
<212>  PRT
<213>  Artificial Sequence

<400>  7
Leu Gly Ser Trp Arg His Trp Arg Gly Gln Glu Gly
1               5                   10

<210>  8
<211>  13
<212>  PRT
<213>  Artificial Sequence

<400>  8
Ser Leu Tyr Lys Lys Val Val Gly Thr Met Ala Ala Gly
1               5                   10

<210>  9
<211>  12
<212>  PRT
<213>  Artificial Sequence

<400>  9
Met Leu Ser Glu Ser Arg Asn Phe Pro Ala Gln Ala
1               5                   10

<210>  10
<211>  12
<212>  PRT
<213>  Artificial Sequence

<400>  10
Glu Arg Ala Pro Thr Gly Arg Trp Gly Arg Arg Gly
1               5                   10

<210>  11
<211>  12
<212>  PRT
<213>  Artificial Sequence

<400>  11
Lys Lys Val Gly Arg Ala Arg Pro Cys Gln Arg Gly
1               5                   10

<210>  12
<211>  12
<212>  PRT
<213>  Artificial Sequence

<400>  12
Ala Pro Arg Ala Pro Arg Gln Arg Ser Arg Asp Gly
1               5                   10

<210>  13
<211>  11
<212>  PRT
<213>  Artificial Sequence

<400>  13
Ser Trp Arg Leu Thr Gly Ser Gly Met Lys Gly
1               5                   10

<210>  14
<211>  12
<212>  PRT
<213>  Artificial Sequence

<400>  14
Trp Arg Pro Gly Arg Arg Gly Pro Ser Ser Gly Gly
1               5                   10

<210>  15
<211>  12
<212>  PRT
<213>  Artificial Sequence

<400>  15
Leu Arg Gly Pro Ser Pro Pro Pro Met Ala Gly Gly
1               5                   10

<210>  16
<211>  12
<212>  PRT
<213>  Artificial Sequence

<400>  16
Trp Arg Pro Gly Arg Arg Gly Pro Ser Ser Gly Gly
1               5                   10

<210>  17
<211>  12
<212>  PRT
<213>  Artificial Sequence

<400>  17
Met Ala Leu Ala Val Arg Val Val Tyr Cys Gly Ala
1               5                   10

<210>  18
<211>  12
<212>  PRT
<213>  Artificial Sequence

<400>  18
Lys Lys Asn Val Glu Ala Ile Gly Leu Leu Gly Gly
1               5                   10

<210>  19
<211>  12
<212>  PRT
<213>  Artificial Sequence

<400>  19
Pro Pro Gly Pro Pro Leu Ser Ser Pro Arg Pro Arg
1               5                   10

<210>  20
<211>  12
<212>  PRT
<213>  Artificial Sequence

<400>  20
Thr Met Asn Asn Glu Glu Asp Leu Leu Arg Ser Leu
1               5                   10

<210>  21
<211>  12
<212>  PRT
<213>  Artificial Sequence

<400>  21
Thr Met Glu Pro Pro Gly Gly Arg Gln Lys Lys Arg
1               5                   10

<210>  22
<211>  12
<212>  PRT
<213>  Artificial Sequence

<400>  22
Gln Glu Arg Gly Pro Thr Trp Asp Lys Asn Leu Arg
1               5                   10

<210>  23
<211>  12
<212>  PRT
<213>  Artificial Sequence

<400>  23
Gly Thr Met Ala Val Leu Arg Gln Arg Pro Gly Arg
1               5                   10

<210>  24
<211>  12
<212>  PRT
<213>  Artificial Sequence

<400>  24
Leu Cys Thr Arg Ser Trp Asp Val Thr Pro Asn Arg
1               5                   10

<210>  25
<211>  12
<212>  PRT
<213>  Artificial Sequence

<400>  25
Ala Asn Gln Pro Ala Gln Cys Arg Lys Thr Arg Ile
1               5                   10

<210>  26
<211>  12
<212>  PRT
<213>  Artificial Sequence

<400>  26
Glu Glu Ala Arg Leu Lys Tyr Asp Lys Ser Arg Ile
1               5                   10

<210>  27
<211>  12
<212>  PRT
<213>  Artificial Sequence

<400>  27
Leu Asn Asp Gly Pro Lys Pro Gly Gln Ser Arg Phe
1               5                   10

<210>  28
<211>  12
<212>  PRT
<213>  Artificial Sequence

<400>  28
Thr Ser Leu Tyr Lys Lys Val Gly Met Gly Arg Lys
1               5                   10

<210>  29
<211>  12
<212>  PRT
<213>  Artificial Sequence

<400>  29
Tyr Lys Lys Val Gly Thr Met Arg Gly Arg Gly Leu
1               5                   10

<210>  30
<211>  12
<212>  PRT
<213>  Artificial Sequence

<400>  30
Val Gly Thr Met Ala Ala Gly Arg Ala Pro Gly Lys
1               5                   10

<210>  31
<211>  12
<212>  PRT
<213>  Artificial Sequence

<400>  31
Lys Lys Val Gly Thr Met Arg Gly Val Gly Tyr Pro
1               5                   10

<210>  32
<211>  12
<212>  PRT
<213>  Artificial Sequence

<400>  32
Glu Gly Pro Leu Trp His Pro Arg Ile Cys Gly Ser
1               5                   10

<210>  33
<211>  12
<212>  PRT
<213>  Artificial Sequence

<400>  33
Glu Met Ala Leu Ser Pro Pro Arg Ser Trp Gly Gln
1               5                   10

<210>  34
<211>  12
<212>  PRT
<213>  Artificial Sequence

<400>  34
Arg Ser Gly Leu Arg Arg Arg Arg His Arg Gly Glu
1               5                   10

<210>  35
<211>  12
<212>  PRT
<213>  Artificial Sequence

<400>  35
Val Ser Gly Ile Met Arg Arg Pro Trp Gly Met Asn
1               5                   10

<210>  36
<211>  12
<212>  PRT
<213>  Artificial Sequence

<400>  36
Lys Arg Val Leu Ile Arg Val Thr Tyr Cys Gly Leu
1               5                   10

<210>  37
<211>  12
<212>  PRT
<213>  Artificial Sequence

<400>  37
Met Ala Leu Ala Val Arg Val Val Tyr Cys Gly Ala
1               5                   10

<210>  38
<211>  35
<212>  PRT
<213>  Artificial Sequence

<400>  38
Pro Ser Ser Pro Val Gln Thr Thr Pro Leu Ser Gln Ala Val Ala Thr
1               5                   10                  15
Pro Ser Arg Ser Ser Ala Ala Ala Ala Ala Ala Leu Asp Leu Ser Gly
            20                  25                  30
Arg Arg Gly
        35

<210>  39
<211>  36
<212>  PRT
<213>  Artificial Sequence

<400>  39
His His Gly Lys Gly Phe Phe Gly Ser Gly His Tyr Thr Ala Tyr Cys
1               5                   10                  15
Tyr Asn Thr Glu Gly Gly Ala Cys Ala Leu Leu Cys Gly Val Gly Asp
            20                  25                  30
Thr Glu Arg Gly
        35

<210>  40
<211>  35
<212>  PRT
<213>  Artificial Sequence

<400>  40
Val Ala Glu Ile Thr Lys Gln Leu Pro Pro Val Val Pro Val Ser Lys
1               5                   10                  15
Pro Gly Ala Leu Arg Arg Ser Leu Ser Arg Ser Met Ser Gln Glu Ala
            20                  25                  30
Gln Arg Gly
        35

<210>  41
<211>  10
<212>  PRT
<213>  Artificial Sequence

<400>  41
Lys Lys Arg Pro Pro Pro Gly Leu Asp Arg
1               5                   10

<210>  42
<211>  10
<212>  PRT
<213>  Artificial Sequence

<400>  42
Ser Val Asn Ser Leu Leu Lys Glu Leu Arg
1               5                   10

<210>  43
<211>  10

<212>  PRT
<213>  Artificial Sequence

<400>  43
His Glu Trp Val Leu Arg Glu Gly Glu Glu
1               5                   10

<210>  44
<211>  10
<212>  PRT
<213>  Artificial Sequence

<400>  44
Glu Tyr Leu Leu Lys Met Ala Thr Glu Glu
1               5                   10

<210>  45
<211>  10
<212>  PRT
<213>  Artificial Sequence

<400>  45
Glu Leu Cys Lys Ser Tyr Arg Arg Leu Gln
1               5                   10

<210>  46
<211>  10
<212>  PRT
<213>  Artificial Sequence

<400>  46
Val Ala Leu Lys Phe Lys Pro Arg Lys His
1               5                   10

<210>  47
<211>  10
<212>  PRT
<213>  Artificial Sequence

<400>  47
Gly Leu Gly Cys Lys Val Leu Arg Arg His
1               5                   10

<210>  48
<211>  6
<212>  PRT
<213>  Artificial Sequence

<400>  48
Arg His Cys Gly Arg Thr
1               5

<210>  49
<211>  6
<212>  PRT
<213>  Artificial Sequence

<400>  49
Arg Ser His Gly Thr Leu
1               5

<210>  50
<211>  6

<212>  PRT
<213>  Artificial Sequence

<400>  50
Arg Phe Arg Gly Leu Arg
1               5

<210>  51
<211>  6
<212>  PRT
<213>  Artificial Sequence

<400>  51
Arg Asn Leu Gly Ile Arg
1               5

<210>  52
<211>  10
<212>  PRT
<213>  Artificial Sequence

<400>  52
Arg Gly Arg Leu Thr Arg Asn Lys Gly Pro
1               5                   10

<210>  53
<211>  10
<212>  PRT
<213>  Artificial Sequence

<400>  53
Ser Leu Phe Arg Lys Arg Asn Lys Gly Lys
1               5                   10

<210>  54
<211>  10
<212>  PRT
<213>  Artificial Sequence

<400>  54
Arg Thr Ala Ala Ser Gly Arg Arg Trp Gly
1               5                   10

<210>  55
<211>  10
<212>  PRT
<213>  Artificial Sequence

<400>  55
Gly Leu Leu Lys Arg Pro Cys Leu Arg Gly
1               5                   10

<210>  56
<211>  10
<212>  PRT
<213>  Artificial Sequence

<400>  56
Gln Arg Lys Leu Gln Arg Thr Ser Arg Gly
1               5                   10

<210>  57
<211>  10

<212>  PRT
<213>  Artificial Sequence

<400>  57
Pro Lys Ser Lys Val Cys Gln Gln Arg Gly
1               5                   10

<210>  58
<211>  10
<212>  PRT
<213>  Artificial Sequence

<400>  58
Lys Arg Leu Leu Lys Gly Ser Gln Tyr Gly
1               5                   10

<210>  59
<211>  12
<212>  PRT
<213>  Artificial Sequence

<400>  59
Pro His Lys Arg Leu Leu Lys Gly Ser Gln Tyr Gly
1               5                   10

<210>  60
<211>  10
<212>  PRT
<213>  Artificial Sequence

<400>  60
Lys Glu Ser Asn Asp Cys Ser Cys Gly Gly
1               5                   10

<210>  61
<211>  10
<212>  PRT
<213>  Artificial Sequence

<400>  61
Asp Cys Val Cys Arg Gly Ser Thr Gly Gly
1               5                   10

<210>  62
<211>  10
<212>  PRT
<213>  Artificial Sequence

<400>  62
Val Ala Pro Arg Ser Arg Asp Glu Arg Gly
1               5                   10

<210>  63
<211>  10
<212>  PRT
<213>  Artificial Sequence

<400>  63
Ala His Gln Leu Gln Ala Leu Arg Arg Gly
1               5                   10

<210>  64
<211>  5

<212>  PRT
<213>  Artificial Sequence

<400>  64
Leu Thr Gly Lys Gly
1               5

<210>  65
<211>  6
<212>  PRT
<213>  Artificial Sequence

<400>  65
Tyr Tyr Cys Phe Phe Gly
1               5

<210>  66
<211>  5
<212>  PRT
<213>  Artificial Sequence

<400>  66
Leu Glu Lys Gly Gly
1               5

<210>  67
<211>  5
<212>  PRT
<213>  Artificial Sequence

<400>  67
Ala Ala His Lys Gly
1               5

<210>  68
<211>  5
<212>  PRT
<213>  Artificial Sequence

<400>  68
Ala Leu Arg Arg Gly
1               5

<210>  69
<211>  5
<212>  PRT
<213>  Artificial Sequence

<400>  69
Gly Gly Ser Gly Gly
1               5

<210>  70
<211>  5
<212>  PRT
<213>  Artificial Sequence

<400>  70
Arg Asp Glu Arg Gly
1               5

<210>  71
<211>  5

<212>  PRT
<213>  Artificial Sequence

<400>  71
Ser Arg Val Lys Gly
1               5

<210>  72
<211>  5
<212>  PRT
<213>  Artificial Sequence

<400>  72
Pro Ala Ser Gly Gly
1               5

<210>  73
<211>  5
<212>  PRT
<213>  Artificial Sequence

<400>  73
Ala Ile His Gly Gly
1               5

<210>  74
<211>  5
<212>  PRT
<213>  Artificial Sequence

<400>  74
Gly Ala Glu Ala Gly
1               5

<210>  75
<211>  5
<212>  PRT
<213>  Artificial Sequence

<400>  75
Gly Ser Thr Gly Gly
1               5

<210>  76
<211>  5
<212>  PRT
<213>  Artificial Sequence

<400>  76
Arg Gly Met Gly Gly
1               5

<210>  77
<211>  5
<212>  PRT
<213>  Artificial Sequence

<400>  77
Leu Val His Ala Gly
1               5

<210>  78
<211>  5

<212>  PRT
<213>  Artificial Sequence

<400>  78
Ser Leu Gln Thr Gly
1               5

<210>  79
<211>  5
<212>  PRT
<213>  Artificial Sequence

<400>  79
Pro Val Pro Gly Gly
1               5

<210>  80
<211>  5
<212>  PRT
<213>  Artificial Sequence

<400>  80
Asn Tyr Lys Ser Gly
1               5

<210>  81
<211>  5
<212>  PRT
<213>  Artificial Sequence

<400>  81
Pro Arg Lys Gln Gly
1               5

<210>  82
<211>  5
<212>  PRT
<213>  Artificial Sequence

<400>  82
Thr Pro Arg Gly Gly
1               5

<210>  83
<211>  5
<212>  PRT
<213>  Artificial Sequence

<400>  83
Gly Cys Ser Gly Gly
1               5

<210>  84
<211>  5
<212>  PRT
<213>  Artificial Sequence

<400>  84
Glu Ala Gln Arg Gly
1               5

<210>  85
<211>  5

<212>  PRT
<213>  Artificial Sequence

<400>  85
Gly Lys Ala Trp Gly
1               5

<210>  86
<211>  5
<212>  PRT
<213>  Artificial Sequence

<400>  86
Pro Ala Gly Gly Gly
1               5

<210>  87
<211>  5
<212>  PRT
<213>  Artificial Sequence

<400>  87
Val Val Leu Tyr Gly
1               5

<210>  88
<211>  5
<212>  PRT
<213>  Artificial Sequence

<400>  88
Leu Thr Leu Lys Gly
1               5

<210>  89
<211>  5
<212>  PRT
<213>  Artificial Sequence

<400>  89
Gly Phe Gln Ser Gly
1               5

<210>  90
<211>  5
<212>  PRT
<213>  Artificial Sequence

<400>  90
Arg Val Gln Trp Gly
1               5

<210>  91
<211>  38
<212>  PRT
<213>  Artificial Sequence

<400>  91
Ser Arg Thr Glu Gly Gln Phe Gly Thr Thr Gln Ser Asn Gly Thr Phe
1               5                   10                  15
Phe Asn Gly Ala Ser Pro Gly Thr Pro Pro Ala Pro Ser Gln His Gln
            20                  25                  30
Gln Ser Leu Thr Ser Leu

        35

<210>  92
<211>  53
<212>  PRT
<213>  Artificial Sequence

<400>  92
Tyr Arg Pro Ile Pro Phe Gln Pro Glu Gly Ala Gly Glu Gly Thr Asp
1               5                   10                  15
Glu Asp Lys Ser Asn Arg Ile Gly Asn Asn Gly Leu Arg Leu Asn Asp
            20                  25                  30
Gly Asn Gly Asn Gly Gln Leu Ala Pro Ser Pro Thr Pro Gln Gly Thr
        35                  40                  45
Glu Ala Val Arg Ala
    50

<210>  93
<211>  37
<212>  PRT
<213>  Artificial Sequence

<400>  93
Arg Lys Phe Ser Asn Asn Pro Gln Pro Asn Ala Ile Ser Asn Gly Thr
1               5                   10                  15
Ser Thr Ser Glu Arg Pro Gly Glu Gly Ala Thr Gln Gly Ile Val Glu
            20                  25                  30
Glu Glu Val Leu Gln
        35

<210>  94
<211>  37
<212>  PRT
<213>  Artificial Sequence

<400>  94
Ile Arg Thr Glu Ser Ala Glu Glu Ala Glu Met Ala Ser Val Pro Asn
1               5                   10                  15
Gly Ser Pro Ser Trp His Pro Gly Ala Ser His Val Val Asn Gly Ala
            20                  25                  30
Ala Gly His Ser Asn
        35

<210>  95
<211>  37
<212>  PRT
<213>  Artificial Sequence

<400>  95
Trp His Glu Ile Glu Met Glu Ser Gly Glu Glu Ala Met Glu Pro Ala
1               5                   10                  15
Asn Glu Thr Gly Asn Thr Leu Asn Gly Ser Pro Ser Trp His Pro Ser
            20                  25                  30
Pro Ser His Val Ile
        35

<210>  96
<211>  37
<212>  PRT
<213>  Artificial Sequence

<400>  96
Asn Arg Thr Asp Phe Ala Gly Glu Glu Asp Glu Met Asp Gly Val Leu

1               5                   10                  15
Asn Gly Ser Pro Ser Trp His Ala Ala Thr Ser His Ile Val Asn Gly
            20                  25                  30
Ala Thr Val His Gln
        35

<210>  97
<211>  37
<212>  PRT
<213>  Artificial Sequence

<400>  97
Asn Arg Thr Asp Thr Ala Ala Glu Ala Glu Met Asp Ser Val Leu Asn
1               5                   10                  15
Gly Ser Pro Ser Trp His Pro Pro Ala Gly His Val Val Asn Gly Ala
            20                  25                  30
Thr Val His Arg Ser
        35

<210>  98
<211>  37
<212>  PRT
<213>  Artificial Sequence

<400>  98
Asn Arg Thr Asp Thr Ala Ala Glu Ala Glu Met Asp Ser Val Leu Asn
1               5                   10                  15
Gly Ser Pro Ser Trp His Pro Pro Ala Gly His Val Val Asn Gly Ala
            20                  25                  30
Ala Val His Arg Ser
        35

<210>  99
<211>  37
<212>  PRT
<213>  Artificial Sequence

<400>  99
Asn Arg Thr Glu Val Leu Glu Gly Ala Glu Ile Pro Ser Thr Val Asn
1               5                   10                  15
Gly Ser Pro Ser Trp His Pro Ala Asp Ser Arg Ala Val Ser Gly Ala
            20                  25                  30
Thr Gly His Ser Ser
        35

<210>  100
<211>  37
<212>  PRT
<213>  Artificial Sequence

<400>  100
Asn Arg Thr Glu Ala Pro Glu Gly Thr Glu Leu Pro Ser Thr Val Asn
1               5                   10                  15
Gly Ser Pro Ser Trp His Pro Ala Asp Ser Arg Ala Gly Ser Gly Ala
            20                  25                  30
Thr Gly His Ser Ser
        35

<210>  101
<211>  37
<212>  PRT
<213>  Artificial Sequence

<400>  101
Asn Arg Thr Glu Ala Pro Glu Gly Thr Glu Ser Glu Arg Glu Thr Pro
1               5                   10                  15
Ser Ala Ile Asn Gly Asn Pro Ser Trp His Leu Ala Asp Ser Pro Ala
            20                  25                  30
Val Asn Gly Ala Thr
        35

<210>  102
<211>  59
<212>  PRT
<213>  Artificial Sequence

<400>  102
Asn Arg Thr Glu Ala Pro Glu Gly Thr Glu Ser Glu Val Glu Thr Pro
1               5                   10                  15
Ser Ala Ile Asn Gly Asn Pro Ser Trp Gln Leu Ala Asp Ser Pro Ala
            20                  25                  30
Ile Asn Gly Ala Thr Gly His Ser Ser Ser Leu Asp Ala Arg Glu Val
        35                  40                  45
Ile Pro Met Ala Ala Val Lys Gln Gln Ala Leu
    50                  55

<210>  103
<211>  37
<212>  PRT
<213>  Artificial Sequence

<400>  103
Asn Arg Thr Glu Ala Pro Glu Gly Thr Glu Ser Asp Met Glu Thr Pro
1               5                   10                  15
Ser Ala Ile Asn Gly Asn Ala Ser Trp His Leu Ala Asp Ser Pro Ala
            20                  25                  30
Val Asn Gly Ala Thr
        35

<210>  104
<211>  37
<212>  PRT
<213>  Artificial Sequence

<400>  104
Asn Arg Thr Glu Ala Pro Glu Gly Thr Gly Pro Glu Met Glu Thr Pro
1               5                   10                  15
Ser Ala Ile Asn Gly Asn Pro Ala Trp His Pro Ala Asp Ser Pro Ala
            20                  25                  30
Val Asn Gly Ala Thr
        35

<210>  105
<211>  37
<212>  PRT
<213>  Artificial Sequence

<400>  105
Asn Arg Thr Glu Ala Pro Glu Gly Thr Glu Ser Asp Met Glu Thr Pro
1               5                   10                  15
Ser Ala Ile Asn Gly Asn Pro Ser Trp His Leu Ala Asp Ser Pro Ala
            20                  25                  30
Val Asn Gly Ala Thr
        35

<210>  106

<211>  37
<212>  PRT
<213>  Artificial Sequence

<400>  106
Asn Arg Thr Glu Ala Pro Glu Gly Thr Glu Ser Glu Ala Glu Thr Pro
1               5                   10                  15
Ser Ala Ile Asn Gly Asn Pro Ser Trp His Leu Ala Asp Ser Pro Ala
            20                  25                  30
Val Asn Gly Ala Thr
        35

<210>  107
<211>  37
<212>  PRT
<213>  Artificial Sequence

<400>  107
Asn Arg Thr Glu Ala Pro Glu Gly Thr Glu Ser Glu Met Glu Thr Pro
1               5                   10                  15
Ser Ala Ile Asn Gly Asn Pro Ser Trp His Leu Ala Asp Ser Pro Pro
            20                  25                  30
Ala Asn Gly Ala Thr
        35

<210>  108
<211>  37
<212>  PRT
<213>  Artificial Sequence

<400>  108
Asn Arg Thr Glu Ala Pro Glu Gly Thr Asp Ser Glu Met Glu Thr Pro
1               5                   10                  15
Ser Ala Ile Asn Gly Asn Pro Ala Trp His Leu Ala Asp Ser Pro Ala
            20                  25                  30
Val Asn Gly Ala Thr
        35

<210>  109
<211>  37
<212>  PRT
<213>  Artificial Sequence

<400>  109
Asn Arg Thr Glu Ala Pro Glu Gly Thr Asp Ser Glu Met Glu Thr Pro
1               5                   10                  15
Ser Ala Ile Asn Gly Asn Pro Ser Trp His Leu Ala Asp Ser Pro Val
            20                  25                  30
Val Asn Gly Ala Thr
        35

<210>  110
<211>  37
<212>  PRT
<213>  Artificial Sequence

<400>  110
Asn Arg Thr Glu Ala Pro Glu Gly Thr Glu Ser Glu Met Glu Thr Pro
1               5                   10                  15
Ser Ala Ile Asn Gly Asn Pro Ser Trp His Leu Ala Asp Ser Pro Ala
            20                  25                  30
Val Asn Gly Ala Thr
        35

<210>  111
<211>  20
<212>  PRT
<213>  Artificial Sequence

<400>  111
Leu Glu Ala Gly Cys Lys Asn Phe Phe Pro Arg Ser Phe Thr Ser Cys
1               5                   10                  15
Gly Ser Leu Glu
            20

<210>  112
<211>  7
<212>  PRT
<213>  Artificial Sequence

<400>  112
Glu Asn Leu Tyr Phe Gln Gly
1               5

<210>  113
<211>  7
<212>  PRT
<213>  Artificial Sequence

<400>  113
Glu Asn Leu Tyr Phe Gln Ser
1               5

<210>  114
<211>  7
<212>  PRT
<213>  Artificial Sequence

<400>  114
Glu Asn Leu Tyr Phe Gln Met
1               5

<210>  115
<211>  7
<212>  PRT
<213>  Artificial Sequence

<400>  115
Asp Asn Leu Tyr Phe Gln Gly
1               5

<210>  116
<211>  7
<212>  PRT
<213>  Artificial Sequence

<400>  116
Gly Asn Leu Tyr Phe Gln Gly
1               5

<210>  117
<211>  7
<212>  PRT
<213>  Artificial Sequence

<400>  117

Tyr Asn Leu Tyr Phe Gln Gly
1               5

<210>  118
<211>  7
<212>  PRT
<213>  Artificial Sequence

<400>  118
Met Asn Leu Tyr Phe Gln Gly
1               5

<210>  119
<211>  7
<212>  PRT
<213>  Artificial Sequence

<400>  119
Trp Asn Leu Tyr Phe Gln Gly
1               5

<210>  120
<211>  7
<212>  PRT
<213>  Artificial Sequence

<400>  120
Cys Asn Leu Tyr Phe Gln Gly
1               5

<210>  121
<211>  7
<212>  PRT
<213>  Artificial Sequence

<400>  121
Arg Asn Leu Tyr Phe Gln Gly
1               5

<210>  122
<211>  7
<212>  PRT
<213>  Artificial Sequence

<400>  122
Leu Asn Leu Tyr Phe Gln Gly
1               5

<210>  123
<211>  7
<212>  PRT
<213>  Artificial Sequence

<400>  123
Arg Asn Leu Tyr Phe Gln Ser
1               5

<210>  124
<211>  7
<212>  PRT
<213>  Artificial Sequence

<400>  124

Ala Asn Leu Tyr Phe Gln Gly
1               5

<210>  125
<211>  7
<212>  PRT
<213>  Artificial Sequence

<400>  125
Gln Asn Leu Ile Phe Gln Gly
1               5

<210>  126
<211>  7
<212>  PRT
<213>  Artificial Sequence

<400>  126
Arg Asn Leu Tyr Phe Gln Cys
1               5

<210>  127
<211>  7
<212>  PRT
<213>  Artificial Sequence

<400>  127
Glu Cys Leu Tyr His Gln Gly
1               5

<210>  128
<211>  7
<212>  PRT
<213>  Artificial Sequence

<400>  128
Glu Arg Leu Tyr Val Gln Met
1               5

<210>  129
<211>  7
<212>  PRT
<213>  Artificial Sequence

<400>  129
Glu Ser Glu Tyr Cys Gln Glu
1               5

<210>  130
<211>  7
<212>  PRT
<213>  Artificial Sequence

<400>  130
Glu Val Met Tyr Ser Gln Ala
1               5

<210>  131
<211>  7
<212>  PRT
<213>  Artificial Sequence

<400>  131

Glu Phe Leu Tyr Ile Gln Asp
1               5

<210>  132
<211>  7
<212>  PRT
<213>  Artificial Sequence

<400>  132
Glu Arg Gly Tyr Gly Gln Val
1               5

<210>  133
<211>  7
<212>  PRT
<213>  Artificial Sequence

<400>  133
Glu Val Trp Tyr Cys Gln Cys
1               5

<210>  134
<211>  7
<212>  PRT
<213>  Artificial Sequence

<400>  134
Glu Val Ala Tyr Gly Gln Lys
1               5

<210>  135
<211>  7
<212>  PRT
<213>  Artificial Sequence

<400>  135
Glu Ser Arg Tyr Val Gln Ser
1               5

<210>  136
<211>  7
<212>  PRT
<213>  Artificial Sequence

<400>  136
Glu Gly Glu Tyr Trp Gln Arg
1               5

<210>  137
<211>  7
<212>  PRT
<213>  Artificial Sequence

<400>  137
Glu Ser Asn Tyr Gly Gln Met
1               5

<210>  138
<211>  21
<212>  PRT
<213>  Artificial Sequence

<400>  138

Gly Ser Gly Gly Glu Asn Leu Tyr Phe Gln Gly Gly Ser Gly Ala Leu
1               5                   10                  15
Ala Pro Tyr Ile Pro
            20

<210>  139
<211>  24
<212>  PRT
<213>  Artificial Sequence

<400>  139
Gly Ser Gly Gly Glu Asn Leu Tyr Phe Gln Gly Gly Ser Gly Asp Arg
1               5                   10                  15
His Asp Ser Gly Leu Asp Ser Met
            20

<210>  140
<211>  55
<212>  PRT
<213>  Artificial Sequence

<400>  140
Gly Ser Gly Gly Glu Asn Leu Tyr Phe Gln Gly Gly Gly Gly Ser Ser
1               5                   10                  15
His Gly Phe Pro Pro Glu Val Glu Glu Gln Asp Asp Gly Thr Leu Pro
            20                  25                  30
Met Ser Cys Ala Gln Glu Ser Gly Met Asp Arg His Pro Ala Ala Cys
        35                  40                  45
Ala Ser Ala Arg Ile Asn Val
    50                  55

<210>  141
<211>  31
<212>  PRT
<213>  Artificial Sequence

<400>  141
Gly Ser Gly Gly Glu Asn Leu Tyr Phe Gln Gly Gly Ser Gly Glu Asn
1               5                   10                  15
Leu Tyr Phe Gln Ser Gly Ser Gly Ala Leu Ala Pro Tyr Ile Pro
            20                  25                  30

<210>  142
<211>  34
<212>  PRT
<213>  Artificial Sequence

<400>  142
Gly Ser Gly Gly Glu Asn Leu Tyr Phe Gln Gly Gly Ser Gly Glu Asn
1               5                   10                  15
Leu Tyr Phe Gln Ser Gly Ser Gly Asp Arg His Asp Ser Gly Leu Asp
            20                  25                  30
Ser Met

<210>  143
<211>  65
<212>  PRT
<213>  Artificial Sequence

<400>  143
Gly Ser Gly Gly Glu Asn Leu Tyr Phe Gln Gly Gly Gly Gly Ser Glu
1               5                   10                  15

Asn Leu Tyr Phe Gln Ser Gly Ser Gly Ser His Gly Phe Pro Pro Glu
            20                  25                  30
Val Glu Glu Gln Asp Asp Gly Thr Leu Pro Met Ser Cys Ala Gln Glu
        35                  40                  45
Ser Gly Met Asp Arg His Pro Ala Ala Cys Ala Ser Ala Arg Ile Asn
    50                  55                  60
Val
65

<210> 144
<211> 31
<212> PRT
<213> Artificial Sequence

<400> 144
Gly Ser Gly Gly Glu Asn Leu Tyr Phe Gln Gly Gly Ser Gly Ala Leu
1               5                   10                  15
Ala Pro Tyr Ile Pro Gly Ser Gly Ala Leu Ala Pro Tyr Ile Pro
            20                  25                  30

<210> 145
<211> 34
<212> PRT
<213> Artificial Sequence

<400> 145
Gly Ser Gly Gly Glu Asn Leu Tyr Phe Gln Gly Gly Ser Gly Asp Arg
1               5                   10                  15
His Asp Ser Gly Leu Asp Ser Met Gly Ser Gly Ala Leu Ala Pro Tyr
            20                  25                  30
Ile Pro

<210> 146
<211> 75
<212> PRT
<213> Artificial Sequence

<400> 146
Gly Ser Gly Gly Glu Asn Leu Tyr Phe Gln Gly Gly Gly Gly Ser Glu
1               5                   10                  15
Asn Leu Tyr Phe Gln Ser Gly Ser Gly Ser His Gly Phe Pro Pro Glu
            20                  25                  30
Val Glu Glu Gln Asp Asp Gly Thr Leu Pro Met Ser Cys Ala Gln Glu
        35                  40                  45
Ser Gly Met Asp Arg His Pro Ala Ala Cys Ala Ser Ala Arg Ile Asn
    50                  55                  60
Val Gly Ser Gly Ala Leu Ala Pro Tyr Ile Pro
65                  70                  75

<210> 147
<211> 41
<212> PRT
<213> Artificial Sequence

<400> 147
Gly Ser Gly Gly Glu Asn Leu Tyr Phe Gln Gly Gly Ser Gly Glu Asn
1               5                   10                  15
Leu Tyr Phe Gln Ser Gly Ser Gly Ala Leu Ala Pro Tyr Ile Pro Gly
            20                  25                  30
Ser Gly Ala Leu Ala Pro Tyr Ile Pro
        35                  40

<210> 148
<211> 44
<212> PRT
<213> Artificial Sequence

<400> 148
Gly Ser Gly Gly Glu Asn Leu Tyr Phe Gln Gly Gly Ser Gly Glu Asn
1               5                   10                  15
Leu Tyr Phe Gln Ser Gly Ser Gly Asp Arg His Asp Ser Gly Leu Asp
            20                  25                  30
Ser Met Gly Ser Gly Ala Leu Ala Pro Tyr Ile Pro
        35                  40

<210> 149
<211> 75
<212> PRT
<213> Artificial Sequence

<400> 149
Gly Ser Gly Gly Glu Asn Leu Tyr Phe Gln Gly Gly Gly Gly Ser Glu
1               5                   10                  15
Asn Leu Tyr Phe Gln Ser Gly Ser Gly Ser His Gly Phe Pro Pro Glu
            20                  25                  30
Val Glu Glu Gln Asp Asp Gly Thr Leu Pro Met Ser Cys Ala Gln Glu
        35                  40                  45
Ser Gly Met Asp Arg His Pro Ala Ala Cys Ala Ser Ala Arg Ile Asn
    50                  55                  60
Val Gly Ser Gly Ala Leu Ala Pro Tyr Ile Pro
65                  70                  75

<210> 150
<211> 237
<212> PRT
<213> Artificial Sequence

<400> 150
Met Gly Glu Ser Leu Phe Lys Gly Pro Arg Asp Tyr Asn Pro Ile Ser
1               5                   10                  15
Ser Thr Ile Cys His Leu Thr Asn Glu Ser Asp Gly His Thr Thr Ser
            20                  25                  30
Leu Tyr Gly Ile Gly Phe Gly Pro Phe Ile Ile Thr Asn Lys His Leu
        35                  40                  45
Phe Arg Arg Asn Asn Gly Thr Leu Leu Val Gln Ser Leu His Gly Val
    50                  55                  60
Phe Lys Val Lys Asn Thr Thr Thr Leu Gln Gln His Leu Ile Asp Gly
65                  70                  75                  80
Arg Asp Met Ile Ile Ile Arg Met Pro Lys Asp Phe Pro Pro Phe Pro
                85                  90                  95
Gln Lys Leu Lys Phe Arg Glu Pro Gln Arg Glu Glu Arg Ile Cys Leu
            100                 105                 110
Val Thr Thr Asn Phe Gln Thr Lys Ser Met Ser Ser Met Val Ser Asp
        115                 120                 125
Thr Ser Cys Thr Phe Pro Ser Ser Asp Gly Ile Phe Trp Lys His Trp
    130                 135                 140
Ile Gln Thr Lys Asp Gly Gln Cys Gly Ser Pro Leu Val Ser Thr Arg
145                 150                 155                 160
Asp Gly Phe Ile Val Gly Ile His Ser Ala Ser Asn Phe Thr Asn Thr
                165                 170                 175
Asn Asn Tyr Phe Thr Ser Val Pro Lys Asn Phe Met Glu Leu Leu Thr
            180                 185                 190
Asn Gln Glu Ala Gln Gln Trp Val Ser Gly Trp Arg Leu Asn Ala Asp
        195                 200                 205
Ser Val Leu Trp Gly Gly His Lys Val Phe Met Val Lys Pro Glu Glu

210                 215                 220
Pro Phe Gln Pro Val Lys Glu Ala Thr Gln Leu Met Asn
225                 230                 235

<210>  151
<211>  20
<212>  DNA
<213>  Artificial Sequence

<400>  151
tcattacaaa caagcacttg                                                   20

<210>  152
<211>  20
<212>  DNA
<213>  Artificial Sequence

<400>  152
taggcatgcg aataattatc                                                   20

<210>  153
<211>  20
<212>  DNA
<213>  Artificial Sequence

<400>  153
ccacatcgct cagacaccat                                                   20

<210>  154
<211>  26
<212>  DNA
<213>  Artificial Sequence

<400>  154
ggcaacaata tccactttac cagagt                                            26

<210>  155
<211>  63
<212>  DNA
<213>  Artificial Sequence

<400>  155
ggttctggtg gtgagaatct gtacttccaa ggtggatctg gagcattggc cccctacatt      60
cca                                                                     63

<210>  156
<211>  72
<212>  DNA
<213>  Artificial Sequence

<400>  156
ggttctggtg gtgagaatct gtacttccaa ggtggatctg gagatcgcca cgattcaggg      60
ctcgattcca tg                                                           72

<210>  157
<211>  165
<212>  DNA
<213>  Artificial Sequence

<400>  157
ggttctggtg gtgagaatct gtacttccaa ggtggaggag gatccagcca tggcttcccg      60
ccggaggtgg aggagcagga tgatggcacg ctgcccatgt cttgtgccca ggagagcggg     120
atggaccgtc accctgcagc ctgtgcttct gctaggatca atgtg                      165

<210>  158
<211>  93
<212>  DNA
<213>  Artificial Sequence

<400>  158
ggttctggtg gtgagaatct gtacttccaa ggtggatctg gagaaaacct ctattttcag      60
tcaggtagtg gtgcattggc cccctacatt cca                                   93

<210>  159
<211>  102
<212>  DNA
<213>  Artificial Sequence

<400>  159
ggttctggtg gtgagaatct gtacttccaa ggtggatctg gagaaaacct ctattttcag      60
tcaggtagtg gtgatcgcca cgattcaggg ctcgattcca tg                        102

<210>  160
<211>  195
<212>  DNA
<213>  Artificial Sequence

<400>  160
ggttctggtg gtgagaatct gtacttccaa ggtggaggag gatccgaaaa cctctatttt      60
cagtcaggta gtggtagcca tggcttcccg ccggaggtgg aggagcagga tgatggcacg     120
ctgcccatgt cttgtgccca ggagagcggg atggaccgtc accctgcagc ctgtgcttct     180
gctaggatca atgtg                                                      195

<210>  161
<211>  93
<212>  DNA
<213>  Artificial Sequence

<400>  161
ggttctggtg gtgagaatct gtacttccaa ggtggatctg gagcattggc cccctacatt      60
ccaggtagtg gtgcccttgc accatatatc ccc                                   93

<210>  162
<211>  102
<212>  DNA
<213>  Artificial Sequence

<400>  162
ggttctggtg gtgagaatct gtacttccaa ggtggatctg gagatcgcca cgattcaggg      60
ctcgattcca tgggatctgg agcattggcc ccctacattc ca                        102

<210>  163
<211>  195
<212>  DNA
<213>  Artificial Sequence

<400>  163
ggttctggtg gtgagaatct gtacttccaa ggtggaggag gatccagcca tggcttcccg      60
ccggaggtgg aggagcagga tgatggcacg ctgcccatgt cttgtgccca ggagagcggg     120
atggaccgtc accctgcagc ctgtgcttct gctaggatca atgtgggatc tggagcattg     180
gccccctaca ttcca                                                      195

<210>  164
<211>  123
<212>  DNA
<213>  Artificial Sequence

<400>  164
ggttctggtg gtgagaatct gtacttccaa ggtggatctg gagaaaacct ctattttcag      60
tcaggtagtg gtgcattggc cccctacatt ccaggtagtg gtgcccttgc accatatatc     120
ccc                                                                   123

<210>  165
<211>  132
<212>  DNA
<213>  Artificial Sequence

<400>  165
ggttctggtg gtgagaatct gtacttccaa ggtggatctg gagaaaacct ctattttcag      60
tcaggtagtg gtgatcgcca cgattcaggg ctcgattcca tgggtagtgg tgcccttgca     120
ccatatatcc cc                                                         132

<210>  166
<211>  225
<212>  DNA
<213>  Artificial Sequence

<400>  166
ggttctggtg gtgagaatct gtacttccaa ggtggaggag gatccgaaaa cctctatttt      60
cagtcaggta gtggtagcca tggcttcccg ccggaggtgg aggagcagga tgatggcacg     120
ctgcccatgt cttgtgccca ggagagcggg atggaccgtc accctgcagc ctgtgcttct     180
gctaggatca atgtgggtag tggtgccctt gcaccatata tcccc                     225

<210>  167
<211>  31
<212>  PRT
<213>  Artificial Sequence

<400>  167
Gly Ser Gly Gly Glu Asn Leu Tyr Phe Gln Gly Gly Ser Gly Ala Leu
1               5                   10                  15
Ala Pro Tyr Ile Pro Asp Arg His Asp Ser Gly Leu Asp Ser Met
            20                  25                  30

<210>  168
<211>  65
<212>  PRT
<213>  Artificial Sequence

<400>  168
Gly Ser Gly Gly Glu Asn Leu Tyr Phe Gln Gly Gly Gly Gly Ser Ser
1               5                   10                  15
His Gly Phe Pro Pro Glu Val Glu Glu Gln Asp Asp Gly Thr Leu Pro
            20                  25                  30
Met Ser Cys Ala Gln Glu Ser Gly Met Asp Arg His Pro Ala Ala Cys
        35                  40                  45
Ala Ser Ala Arg Ile Asn Val Gly Ser Gly Ala Leu Ala Pro Tyr Ile
    50                  55                  60
Pro
65

<210>  169
<211>  93
<212>  DNA
<213>  Artificial Sequence

<400>  169
ggttctggtg gtgagaatct gtacttccaa ggtggatctg gagcattggc cccctacatt      60
ccagatcgcc acgattcagg gctcgattcc atg                                   93

<210>  170
<211>  195
<212>  DNA
<213>  Artificial Sequence

<400>  170
ggttctggtg gtgagaatct gtacttccaa ggtggaggag gatccagcca tggcttcccg        60
ccggaggtgg aggagcagga tgatggcacg ctgcccatgt cttgtgccca ggagagcggg       120
atggaccgtc accctgcagc ctgtgcttct gctaggatca atgtgggatc tggagcattg       180
gccccctaca ttcca                                                        195

## Description

### Technical Field

The present invention belongs to the field of biotechnology. Specifically, the present invention relates to a proteolysis-targeting virus and uses thereof. The present invention also provides methods for preparing the virus.

### Background Art

Influenza is an acute disease that spreads through the respiratory tract and is caused by influenza viruses, and can seasonally infect poultry, mammals and humans. Influenza viruses can be divided into three types: A, B and C, wherein influenza A (also known as type A) virus outbreaks most frequently. Influenza A virus belongs to the family orthomyxoviridae, having a genome consisting of 8 independent single-stranded RNA fragments in total that encode 10 proteins: hemagglutinin (HA); matrix protein (M), which is divided into M1 and M2; neuraminidase (NA); nucleocapsid protein (NP); non-structural protein (NS), including NS1 and NEP; and three polymerases, PB1, PB2 and PA. Each of these proteins has important biological functions for influenza virus. Influenza A virus is classified into different subtypes according to the antigenic differences of their major surface antigens HA and NA. At present, 18 subtypes of HA protein and 11 subtypes of NA protein have been found. Influenza A virus pandemics can cause extremely high morbidity and mortality, posing a serious threat to human health (W.H.O. 2003; Coleman 2007). Influenza A virus mainly caused three influenza pandemics in the twentieth century, that is, H1N1 in 1918, H2N2 in 1957, and H3N2 in 1968, resulting in a total of approximately 50 million deaths (Kilbourne 2006; Taubenberger, Hultin et al. 2007). Influenza A in 2009 was also caused by the H1N1 influenza virus (Dawood, Jain et al. 2009; Zimmer and Burke 2009), and its rapid spread had attracted worldwide concern. Statistically, on average, 300,000-500,000 people die of influenza worldwide each year (Fiore, Shay et al. 2007).

Since the discovery of influenza, scientists have been working on the prevention and control of influenza viruses. Vaccination is currently the most beneficial means to preventing influenza and controlling its spread. In the late 1930s, influenza virus vaccines began to be used in humans. Current influenza virus vaccines are classified into the following categories: inactivated virus vaccine, attenuated virus vaccine, DNA vaccine, subunit vaccine, recombinant virus vector vaccine and virus-like particle vaccine.

The currently applied inactivated influenza vaccines are trivalent vaccines, including H1N1, H3N2 and influenza B virus vaccines. Years of clinical application have shown that inactivated influenza vaccines have a good immune effect and safety, and can stimulate the body to produce corresponding antibodies after vaccination, but their disadvantage is incompetence in stimulating the production of secretory immunoglobulin (sIgA). In addition, antigenic variation will occur when influenza viruses are passaged in chicken embryos, and most chicken bodies carry a variety of viruses, and thus there is a possibility of vaccine contamination by these viruses. Since newly epidemic antigenic variants must be able to replicate efficiently in chicken embryos to make it possible to produce a large amount of vaccines. As the wild type viruses used for vaccine preparation grow in eggs, their immunogenicity is altered or reduced to some extent. If the vaccine strains produced do not match the currently epidemic strains, the immunoprotective effect will be lost. A major advance in recent years is to replace chicken embryos with mammalian cells for culture. Mammalian cells, mainly MDCK cells and Vero cells, have the advantages of no contamination by exogenous factors, ease of large scale production, antigenic stability and etc. The influenza vaccines cultured in mammalian cells have a better immunogenicity, mild post-vaccination adverse reactions and higher safety. Good experimental results were achieved in the research phase. However, there are still some problems that cannot be solved, and clinical use has not yet been seen.

Attenuated live vaccine mainly includes five types: temperature-sensitive vaccine, reassortant vaccine, cold-adapted attenuated live influenza vaccine, reverse genetic technology vaccine and replication-defective influenza vaccine. Cold-adapted attenuated live influenza vaccine now has been successfully developed. This vaccine is a strain of influenza virus with reduced virulence and capability of growing at an optimal adaptive temperature. This virus can replicate only at about 25°C and cannot be passaged at 37°C. Therefore, its infection site is limited to the upper respiratory tract, and there are no clinically significant influenza symptoms. By using genetic recombination between an attenuated strain with a currently epidemic strain, obtained is a recombinant virus carrying the fragments of the attenuated strain and the HA and NA genes of the epidemic strain. Several studies have confirmed that cold-adapted strains have a good stability of biological properties. Both attenuated influenza vaccine and inactivated vaccine have an antibody positive conversion rate of 50% to 70% after immunization, which can effectively control influenza epidemic. Cold-adapted attenuated live vaccine has been used in Russia and will be also approved for use in the United States. It has certain advantages over inactivated vaccine in terms of vaccination route and immunization effect, such as immunization by intranasal spray or drop. Cold-adapted attenuated live vaccine replicates in the upper respiratory tract and can induce sIgA in the mucosa and systemic humoral and cellular immune responses, resulting in a broader and longer-lasting protection than inactivated vaccine. However, cold-adapted attenuated live vaccine can be genetically reassorted with other influenza viruses to produce toxic reassortant virus strains, and interference may occur in bivalent or trivalent cold-adapted attenuated live vaccines.

An ideal vaccine should have the following features: (i) strong immunogenicity, (ii) low toxic response, (iii) genetic stability, and (iv) ability to rapid acquisition of antigenicity that is consistent with the epidemic strain during the epidemic period. In view of the advantages of live vaccines over inactivated vaccines and the safety considerations of live vaccines, use of mammalian cells in producing new live influenza virus vaccines with controllable replication, genetic stability, safety and effectiveness, and improving the safety and effectiveness of vaccines, will promote the rapid development of influenza vaccines.

### Ubiquitin-proteasome system

Protein degradation is critical for the maintenance of normal cellular functions (e.g., proliferation, differentiation and death). Degradation of proteins in eukaryotic cells mainly comprise the lysosomal pathway, specific organelle hydrolysis system, cell membrane surface hydrolysis system, caspase protease system, and highly conserved ubiquitin-proteasome pathway (UPP). The ubiquitin-proteasome pathway is an ATP-dependent protein degradation pathway in the cytoplasm and cell nucleus, which is different from the lysosomal pathway and degrades efficiently the proteins produced by cells with a high selectivity under stress and non-stress conditions, especially functional proteins with a short half-life, oncogene products, denatured and allosteric proteins and etc. It also degrades intracellular structural proteins under stress conditions. The discovery of this protein hydrolysis pathway is considered to be a turning point in the study of cell cycle and cell malignant transformation, which is a very important regulating factor of cell functions.

The ubiquitin-proteasome system consists of ubiquitin, ubiquitin-activating enzyme (E1), ubiquitin-conjugating enzyme (E2), and unbiquitin-protein ligase (E3) and proteasome. Ubiquitin is a highly conserved, small molecule protein composed of 76 amino acids with a molecular weight of 8.45 k. It is widely distributed from cell nucleus to cytoplasm, from germ cells to various somatic cells, which is highly expressed in actively proliferating tumor cells and embryonic cells. The carboxyl group of glycine (Gly) at the C-terminus of ubiquitin, with the assistance of ubiquitin ligase, binds to the α- or ε- amino group of lysine (Lys) in a target protein to be degraded, and forms a polyubiquitin chain, so that the target protein is recognized and hydrolyzed by the proteasome. Ubiquitin-activating enzyme (E1) catalyzes the binding of ubiquitin to protein substrate. Upon the ubiquitin pathway is initiated, with the participation of ATP, the C-terminus of glycine residue of ubiquitin is linked to the activation site, cysteine (Cys) of ubiquitin-conjugating enzyme (E2), and then ubiquitin is activated. After ubiquitin is activated, activated ubiquitin is transferred by ubiquitin-conjugating enzyme (E2) to the activation site, cysteine residue of the ubiquitin-conjugating enzyme. Ubiquitin protein ligase (E3) plays an important role in determining the selectivity of ubiquitin-mediated degradation of substrate proteins. E3 has affinity for enzyme substrate and needs to be linked to E2 to transfer ubiquitin from E2 to a target protein. After ubiquitin binds to enzyme substrate, a polyubiquitin chain is usually formed. In this chain, the C-terminus of each ubiquitin monomer is linked to the specific lysine residue of the previous ubiquitin. Proteasome is a key enzyme that catalyzes the degradation of conjugates of ubiquitin and substrate protein, which includes 20S proteasome and 26S proteasome. The α subunit of 20S is mainly used for substrate recognition, while the β subunit is mainly used for substrate degradation. The role of 26S proteasome may be related to the production of free peptides, free ubiquitin or reusable ubiquitin. Therefore, the role of the ubiquitin-proteasome system consists of two consecutive steps: (1) covalent binding of the ubiquitin molecule to a target protein; and (2) polyubiquitinated protein is degraded by 26S proteasome, and ubiquitin is reactivated at the same time. This process can be achieved only with the assistance of three enzymes, E1, E2, and E3, throughout the plasma membrane system of entire cell, including cell membrane, endoplasmic reticulum to nuclear membrane, and etc. The ubiquitin-proteasome pathway is an important protein regulatory system in eukaryotic cells, which is involved in the regulation of various cellular physiological processes, such as cell cycle progression, cell proliferation and differentiation, signal transduction, and etc. Therefore, ubiquitination degradation of intracellular proteins is an important post-transcriptional modification of proteins.

In short, cells in an organism constantly strive to maintain proper protein levels, and they are producing and degrading thousands of proteins every moment. The key factor in maintaining protein equilibrium is a small protein called ubiquitin. Upon being linked to proteins, it will cause these proteins to be transported to the proteasome for degradation. With this principle, researchers have developed protein degraders based on Proteolysis-Targeting Chimeras (PROTACs) technology. That is, researchers have designed a small molecule with two active termini, one for binding to a targeted protein and the other for binding to a protein called E3 ubiquitin ligase. This bifunctional small molecule can force ubiquitin to bind to targeted proteins, transporting them to the waste disposal site in cells. After the proteins are degraded, they can continue to target other proteins, so as to rapidly reduce the levels of unwanted proteins.

However, there is currently no report on use of this technology for virus modification.

### Summary of the Invention

In view of the deficiencies in the prior art, the present invention provides a proteolysis-targeting virus comprising a molecule that can be recognized by the ubiquitin-proteasome system and a linker. The present invention also provides methods for preparing the virus and uses of the virus.

In the present application, the term "proteolysis-targeting molecule" refers to a variety of molecules, such as polypeptides, amino acid sequences, proteins or other molecules, which can be recognized by the ubiquitin-proteasome system. The applicants have found that by introducing a nucleotide sequence encoding a proteolysis-targeting molecule into the genome of a virus, the proteolysis-targeting molecule can replicate with the replication of viral genome, and be expressed and fused to the viral protein with the translation of viral protein, resulting in a virus that is site-specifically modified by the proteolysis-targeting molecule, i.e., a proteolysis-targeting chimeric virus (PROTAC virus).

Since the ubiquitin-proteasome system is widely present in host cells, in order to avoid the degradation of PROTAC virus by the intracellular ubiquitin-proteasome system during the preparation process, the inventors introduced a selectively cleavable linker between the viral protein and the proteolysis-targeting molecule, which can be cleaved in a specific artificially modified cell line, and thus the viral protein is separated from the proteolysis-targeting molecule, and the viral protein will not be degraded by the ubiquitin-proteasome system and thus will be retained. Accordingly, PROTAC virus can efficiently replicate and be produced in a large quantity in this specific artificially modified cell line. However, in normal cells, the ubiquitin-proteasome system will recognize the proteolysis-targeting molecule fused to the viral protein, and thus degrade the viral protein, and the replication ability of the virus will be diminished or even completely lost, so that PROTAC virus has a very high safety. In addition, PROTAC virus can be further modified, for example, by introducing some immunopotentiators to the specific region of the viral proteins or specific amino acid to obtain a virus with improved properties, thereby obtaining a PROTAC virus with an enhanced immunogenicity.

Another object of the present invention is to introduce into influenza virus genome a nucleotide sequence encoding a proteolysis-targeting molecule that can be conditionally cleaved, such that the influenza virus can replicate only in a specific virus production system capable of cleaving off or inactivating the proteolysis-targeting molecule. By use of the dependence of influenza virus on this specific virus production system, a large quantity of influenza viruses can be prepared in this system. Since the ubiquitin-proteasome pathway exists in normal cells of humans and animals, etc., the proteolysis-targeting molecules expressed and fused to the viral proteins can be recognized, and thereby the viral proteins are degraded, so that the prepared influenza virus cannot replicate and reproduce in animals and humans, increasing the safety of the virus, thereby making this influenza virus a true live influenza virus vaccine. In addition, the ubiquitin-proteasome system in animals and humans can recognize a variety of proteolysis-targeting molecules, so different types and different numbers of proteolysis-targeting molecules can be introduced into the viral proteins, and there are various linkers that can be selectively cleaved, so different types and different numbers of linkers can be introduced to achieve selective cleavage of the proteolysis-targeting molecule. These different types and different numbers of proteolysis targeting molecules and linkers can be combined randomly, so as to guarantee the preparation of viral vaccines with different replication efficiencies and different degrees of attenuation, which are critical for the production efficiency and immunogenicity of influenza virus vaccines.

The principles of this proteolysis targeting virus are that: (1) the proteolysis-targeting molecule introduced into a specific site of viral protein can be recognized by the ubiquitin-proteasome system in normal host cells, thereby degrading and inactivating the associated viral protein; (2) the proteolysis-targeting molecule introduced into a specific site of viral protein can be inhibited in a specific virus production system or separated from the viral protein by selective cleavage of the linker, thus avoiding or reducing degradation of the viral protein by the ubiquitin-proteasome pathway; and (3) the proteolysis-targeting molecule introduced into a specific site of viral protein cannot be inhibited in normal host cells, or the linker linking the proteolysis-targeting molecule to the viral protein cannot be cleaved in normal host cells. Therefore, the prepared virus can be recognized and degraded by the ubiquitin-proteasome pathway in cells of hosts, such as animals and humans, and thus the replication capacity is reduced or even completely lost, increasing the safety of the virus.

In one aspect, the present invention provides a proteolysis-targeting virus comprising one or more proteolysis-targeting molecules at one or more different sites of viral protein thereof that can be recognized by the ubiquitin-proteasome system, and the viral protein is linked to the proteolysis-targeting molecules by one or more linkers, wherein the linker(s) is/are capable of being selectively cleaved.

Preferably, the sites is the C-terminus and/or N-terminus of the protein, that is, the C-terminus and/or N-terminus of gene fragment of the virus.

In the proteolysis-targeting virus according to the present invention, the plurality of proteolysis-targeting molecules may be the same proteolysis-targeting molecules, or a combination of different proteolysis-targeting molecules, and the plurality of linkers may be the same linkers, or a combination of different linkers.

Preferably, the proteolysis-targeting molecule is any of the amino acid sequences selected from those as shown in SEQ ID NO: 1-110:

| SQE ID NO: | amino acid sequence of proteolysis-targeting molecule |
|---|---|
| 1 | ALAPYIP |
| 2 | DRHDSGLDSM |
| 3 | SHGFPPEVEEQDDGTLPMSCAQESGMDRHPAACASARINV |
| 4 | FVNQHLCGSHLVEALYLVCGERGFFYTPKA |
| 5 | DSGXXS (wherein X represents any amino acid) |
| 6 | DRHDSGXXSM (wherein X represents any amino acid) |
| 7 | LGSWRHWRGQEG |
| 8 | SLYKKVVGTMAAG |
| 9 | MLSESRNFPAQA |
| 10 | ERAPTGRWGRRG |
| 11 | KKVGRARPCQRG; |
| 12 | APRAPRQRSRDG; |
| 13 | SWRLTGSGMKG; |
| 14 | WRPGRRGPSSGG; |
| 15 | LRGPSPPPMAGG; |
| 16 | WRPGRRGPSSGG; |
| 17 | MALAVRVVYCGA; |
| 18 | KKNVEAIGLLGG; |
| 19 | PPGPPLSSPRPR; |
| 20 | TMNNEEDLLRSL; |
| 21 | TMEPPGGRQKKR; |
| 22 | QERGPTWDKNLR; |
| 23 | GTMAVLRQRPGR; |
| 24 | LCTRSWDVTPNR; |
| 25 | ANQPAQCRKTRI; |
| 26 | EEARLKYDKSRI; |
| 27 | LNDGPKPGQSRF; |
| 28 | TSLYKKVGMGRK; |
| 29 | YKKVGTMRGRGL; |
| 30 | VGTMAAGRAPGK; |
| 31 | KKVGTMRGVGYP; |
| 32 | EGPLWHPRICGS; |
| 33 | EMALSPPRSWGQ; |
| 34 | RSGLRRRRHRGE; |
| 35 | VSGIMRRPWGMN; |
| 36 | KRVLIRVTYCGL; |
| 37 | MALAVRVVYCGA; |
| 38 | PSSPVQTTPLSQA V ATPSRSSAAAAAALDLSGRRG; |
| 39 | HHGKGFFGSGHYTAYCYNTEGGACALLCGVGDTERG; |
| 40 | VAEITKQLPPVVPVSKPGALRRSLSRSMSQEAQRG; |
| 41 | KKRPPPGLDR; |
| 42 | SVNSLLKELR; |
| 43 | HEWVLREGEE; |
| 44 | EYLLKMATEE; |
| 45 | ELCKSYRRLQ; |
| 46 | VALKFKPRKH; |
| 47 | GLGCKVLRRH; |
| 48 | RHCGRT; |
| 49 | RSHGTL; |
| 50 | RFRGLR; |
| 51 | RNLGIR; |
| 52 | RGRLTRNKGP; |
| 53 | SLFRKRNKGK; |
| 54 | RTAASGRRWG; |
| 55 | GLLKRPCLRG; |
| 56 | QRKLQRTSRG; |
| 57 | PKSKVCQQRG; |
| 58 | KRLLKGSQYG; |
| 59 | PHKRLLKGSQYG; |
| 60 | KESNDCSCGG; |
| 61 | DCVCRGSTGG; |
| 62 | VAPRSRDERG; |
| 63 | AHQLQALRRG; |
| 64 | LTGKG; |
| 65 | YYCFFG; |
| 66 | LEKGG; |
| 67 | AAHKG; |
| 68 | ALRRG; |
| 69 | GGSGG; |
| 70 | RDERG; |
| 71 | SRVKG; |
| 72 | PASGG; |
| 73 | AIHGG; |
| 74 | GAEAG; |
| 75 | GSTGG; |
| 76 | RGMGG; |
| 77 | LVHAG; |
| 78 | SLQTG; |
| 79 | PVPGG; |
| 80 | NYKSG; |
| 81 | PRKQG; |
| 82 | TPRGG; |
| 83 | GCSGG; |
| 84 | EAQRG; |
| 85 | GKAWG; |
| 86 | PAGGG; |
| 87 | VVLYG; |
| 88 | LTLKG; |
| 89 | GFQSG; |
| 90 | RVQWG; |
| 91 | SRTEGQFGTTQSNGTFFNGASPGTPPAPSQHQQSLTSL; |
| 92 | YRPIPFQPEGAGEGTDEDKSNRIGNNGLRLNDGNGNGQLA PSPTPQGTEAVRA; |
| 93 | RKFSNNPQPNAISNGTSTSERPGEGATQGIVEEEVLQ; |
| 94 | IRTESAEEAEMASVPNGSPSWHPGASHVVNGAAGHSN; |
| 95 | WHEIEMESGEEAMEPANETGNTLNGSPSWHPSPSHVI; |
| 96 | NRTDFAGEEDEMDGVLNGSPSWHAATSHIVNGATVHQ; |
| 97 | NRTDTAAEAEMDSVLNGSPSWHPPAGHVVNGATVHRS; |
| 98 | NRTDTAAEAEMDSVLNGSPSWHPPAGHVVNGAA VHRS; |
| 99 | NRTEVLEGAEIPSTVNGSPSWHPADSRAVSGATGHSS; |
| 100 | NRTEAPEGTELPSTVNGSPSWHPADSRAGSGATGHSS; |
| 101 | NRTEAPEGTESERETPSAINGNPSWHLADSPAVNGAT; |
| 102 | NRTEAPEGTESEVETPSAINGNPSWQLADSPAINGATGHSS SLDAREVIPMAAVKQQAL; |
| 103 | NRTEAPEGTESDMETPSAINGNASWHLADSPAVNGAT; |
| 104 | NRTEAPEGTGPEMETPSAINGNPAWHPADSPAVNGAT; |
| 105 | NRTEAPEGTESDMETPSAINGNPSWHLADSPAVNGAT; |
| 106 | NRTEAPEGTESEAETPSAINGNPSWHLADSPAVNGAT; |
| 107 | NRTEAPEGTESEMETPSAINGNPSWHLADSPPANGAT; |
| 108 | NRTEAPEGTDSEMETPSAINGNPAWHLADSPAVNGAT; |
| 109 | NRTEAPEGTDSEMETPSAINGNPSWHLADSPVVNGAT; |
| 110 | NRTEAPEGTESEMETPSAINGNPSWHLADSPAVNGAT; |

Preferably, the linker is selected from any molecules, preferably amino acid sequences, that can be selectively cleaved, such as molecules that can be selectively cleaved by tobacco etch virus protease (TEVp), cleavable molecules that are sensitive to thrombin (restriction enzyme site is LVPR^{∗}GS, e.g. SEQ ID NO: 111 LEAGCKNFFPRSFTSCGSLE), molecules that are cleavable by coagulation factor Xa (restriction enzyme site is IDGR^{∗}), molecules that are cleavable by enterokinase (restriction enzyme site is DDDDK^{∗}), molecules that are cleavable by 3C protease (restriction enzyme site is ETLFQ^{∗}GP), molecules or sequences that are cleavable by SUMO protease, molecules that are cleavable by bacterial gelatinase (e.g., GPLGV), and self-cleavable linkers, preferably those selected from 2A short peptides, such as P2A derived from porcine teschovirus-1, E2A derived from equine rhinitis A virus, F2A derived from foot and mouth disease virus (FMDV), self-cleavable T2A, and etc.

More preferably, the linker is any sequence E-Xₐₐ-Xₐₐ-Y-Xₐₐ-Q-(G/S/M) that can be recognized and cleaved by TEVp (the cleavage occurs between Q and G, or between Q and S, or between Q and M; Xₐₐ represents that it can be any amino acid; herein, the sequence is referred to as the tobacco etch virus protease cleavage sequence and the cleavage site is referred to as the tobacco etch virus protease cleavage site, TEVcs), and specifically any of those selected from the amino acid sequences as shown in SEQ ID NO: 112-137.

| SEQ ID NO: | amino acid sequence | SEQ ID NO: | amino acid sequence |
|---|---|---|---|
| 112 | ENLYFQG | 125 | QNLIFQG |
| 113 | ENLYFQS | 126 | RNLYFQC |
| 114 | ENLYFQM | 127 | ECLYHQG |
| 115 | DNLYFQG | 128 | ERLYVQM |
| 116 | GNLYFQG | 129 | ESEYCQE |
| 117 | YNLYFQG | 130 | EVMYSQA |
| 118 | MNLYFQG | 131 | EFLYIQD |
| 119 | WNLYFQG | 132 | ERGYGQV |
| 120 | CNLYFQG | 133 | EVWYCQC |
| 121 | RNLYFQG | 134 | EVAYGQK |
| 122 | LNLYFQG | 135 | ESRYVQS |
| 123 | RNLYFQS | 136 | EGEYWQR |
| 124 | ANLYFQG | 137 | ESNYGQM |

Preferably, a flexible connector is also comprised between the proteolysis-targeting molecule and the linker.

More preferably, the proteolysis-targeting molecule, linker and flexible connector are linked according to the following mode:
flexible connector-linker-flexible connector-proteolysis-targeting molecule.

Further preferably, the flexible connector-linker-flexible connector-proteolysis-targeting molecule is any of amino acid sequences selected from those as shown in SEQ ID NO: 138-149, 167 and 168.

| SEQ ID NO: | amino acid sequence of flexible connector-linker-flexible connector-proteolysis-targeting molecule |
|---|---|
| 138 | GSGGENLYFQGGSGALAPYIP (named as PTD1) |
| 139 | GSGGENLYFQGGSGDRHDSGLDSM (named as PTD2) |
| 140 | GSGGENLYFQGGGGSSHGFPPEVEEQDDGTLPMSCAQESGM DRHPAACASARINV (named as PTD3) |
| 141 | GSGGENLYFQGGSGENLYFQSGSGALAPYIP |
| 142 | GSGGENLYFQGGSGENLYFQSGSGDRHDSGLDSM |
| 143 | GSGGENL YFQGGGGSENL YFQSGSGSHGFPPEVEEQDDGTLP |
| 144 | MSCAQESGMDRHPAACASARINV GSGGENLYFQGGSGALAPYIPGSGALAPYIP (named as PTD4) |
| 145 | GSGGENLYFQGGSGDRHDSGLDSMGSGALAPYIP (named as PTD5) |
| 146 | GSGGENLYFQGGGGSENLYFQSGSGSHGFPPEVEEQDDGTLP MSCAQESGMDRHPAACASARINVGSGALAPYIP |
| 147 | GSGGENLYFQGGSGENLYFQSGSGALAPYIPGSGALAPYIP (named as PTD8) |
| 148 | GSGGENLYFQGGSGENLYFQSGSGDRHDSGLDSMGSGALAP YIP (named as PTD9) |
| 149 | GSGGENLYFQGGGGSENLYFQSGSGSHGFPPEVEEQDDGTLP MSCAQESGMDRHPAACASARINVGSGALAPYIP (named as PTD10) |
| 167 | GSGGENLYFQGGSGALAPYIPGSGDRHDSGLDSM (named as PTD6) |
| 168 | GSGGENLYFQGGGGSSHGFPPEVEEQDDGTLPMSCAQESGM DRHPAACASARINVGSGALAPYIP (named as PTD7) |

Preferably, the virus is selected from influenza virus, HIV, hand-foot-mouth virus, coxsackie virus, hepatitis C virus HCV, hepatitis B virus HBV, hepatitis A virus, hepatitis D virus, hepatitis E virus, EB virus, human papilloma virus HPV, herpes simplex virus HSV, cytomegalovirus, varicella-zoster virus, vesicular stomatitis virus, respiratory syncytial virus RSV, dengue virus, Ebola virus, Marburg virus, Zika virus, SARS, Middle East respiratory syndrome virus, rotavirus, rabies virus, measles virus, adenovirus, poliovirus, echovirus, encephalitis B virus, forest encephalitis virus, hantavirus, novel enterovirus, rubella virus, mumps virus, parainfluenza virus, blue ear virus, swine fever virus, foot-and-mouth disease virus, microvirus, prion virus, smallpox virus, tobacco mosaic virus, adeno-associated virus, phage, herpes virus, West Nile virus, Norovirus, human boca virus, coronavirus and novel coronavirus SARS-CoV-2, and more preferably, the virus is influenza virus or novel coronavirus SARS-CoV-2.

Preferably, the virus is a modified virus, which is modified or comprises a combination of several insertion modifications.

According to a specific embodiment of the present invention, provided is a proteolysis-targeting influenza virus (PROTAC influenza virus), comprising one or more proteolysis-targeting molecules at one or more different sites of viral protein thereof that can be recognized by the ubiquitin-proteasome system, and the viral protein is linked to the proteolysis-targeting molecule by a linker, wherein the linker is E-Xₐₐ-Xₐₐ-Y-Xₐₐ-Q-(G/S), which can be specifically recognized and cleaved by tobacco etch virus protease (TEVp).

Preferably, one or more of PA, PB1, PB2, NP, HA, NA, M1, M2, NS1, and NEP proteins of influenza virus comprise the proteolysis-targeting molecule and linker, that is, one or more of PA, PB1, PB2, NP, HA, NA, M1, M2, NS1, and NEP gene fragments of influenza virus genome comprise a nucleotide sequence encoding the proteolysis-targeting molecule and linker, i.e., the proteolysis-targeting molecule and linker are site-specifically modified into the corresponding site of one or more of PA, PB1, PB2, NP, HA, NA, M1, M2, NS1, and NEP proteins of influenza virus.

Preferably, both of PA and PB2 of influenza virus comprise one or more proteolysis-targeting molecules and linkers.

Both of PA protein and PB1 protein of influenza virus comprise one or more proteolysis-targeting molecules and linkers.

Both of PB2 protein and PB1 protein of influenza virus comprise one or more proteolysis-targeting molecules and linkers.

All of PA protein, PB2 protein, and PB1 protein of influenza virus comprise one or more proteolysis-targeting molecules and linkers.

All of PA protein, PB2 protein, PB1 protein, and M1 protein of influenza virus comprise one or more proteolysis-targeting molecules and linkers.

All of PA protein, PB2 protein, PB1 protein, M1 protein, and NP protein of influenza virus comprise one or more proteolysis-targeting molecules and linkers.

All of PB2 protein, PB1 protein, and M1 protein of influenza virus comprise one or more proteolysis-targeting molecules and linkers.

Both of PA protein and M1 protein of influenza virus comprise one or more proteolysis-targeting molecules and linkers.

Both of PB1 protein and M1 protein of influenza virus comprise one or more proteolysis-targeting molecules and linkers.

Both of PB2 protein and M1 protein of influenza virus comprise one or more proteolysis-targeting molecules and linkers.

All of PB2 protein, PB1 protein, M1 protein, and NS1 protein of influenza virus comprise one or more proteolysis-targeting molecules and linkers.

All of PB2 protein, PB1 protein, M1 protein, and NEP protein of influenza virus comprise one or more proteolysis-targeting molecules and linkers.

Both of NS1 protein and NEP protein of influenza virus comprise one or more proteolysis-targeting molecules and linkers.

Preferably, the proteolysis-targeting virus is a proteolysis-targeting coronavirus, comprising one or more proteolysis-targeting molecules at one or more different sites of viral protein thereof that can be recognized by the ubiquitin-proteasome system, and the viral protein is linked to the proteolysis-targeting molecule by a linker, wherein the linker is E-Xₐₐ-Xₐₐ-Y-Xₐₐ-Q-(G/S/M), which can be specifically recognized and cleaved by tobacco etch virus protease.

Preferably, the virus is novel coronavirus SARS-CoV-2.

More preferably, one or more of spike (S) protein, envelope (E) glycoprotein, membrane (M) glycoprotein, nucleocapsid (N) protein, non-structural protein 1 (nsp1), non-structural protein 2, non-structural protein 3, non-structural protein 4, non-structural protein 5, non-structural protein 6, non-structural protein 7, non-structural protein 8, non-structural protein 9, non-structural protein 10, non-structural protein 11, non-structural protein 12, non-structural protein 13, non-structural protein 14, non-structural protein 15, non-structural protein 16, 3a protein, 3b protein, 6 protein, 7a protein, 7b protein, 8a protein, 8b protein, 9b protein, 3C-like proteinase, leader protein, 2'-O-ribose methyltransferase, endonuclease (endoRNAse), 3'- to 5'- exonuclease, helicase, RNA-dependent RNA polymerase, orfla polyprotein, ORF10 protein, ORF8 protein, ORF7a protein, ORF6 protein, and ORF3a protein of coronavirus comprise one or more proteolysis-targeting molecules and linkers.

Preferably, the proteolysis-targeting virus is a proteolysis-targeting HIV virus, comprising one or more proteolysis-targeting molecules at one or more different sites of viral protein thereof that can be recognized by the ubiquitin-proteasome system, and the viral protein is linked to the proteolysis-targeting molecule by a linker, wherein the linker is E-Xₐₐ-Xₐₐ-Y-Xₐₐ-Q-(G/S/M), which can be specifically recognized and cleaved by tobacco etch virus protease.

Preferably, the virus is an HIV virus.

Still preferably, one or more of Gag polyprotein, pol polyprotein, gp160, HIV trans-activator of transcription (Tat), regulator of expression of virion protein (Rev), viral negative factor (Nef), lentiviral protein R (Vpr), viral infectivity factor (Vif), viral protein U (Vpu), matrix protein (MA, p17), capsid protein (CA, p24), spacer peptide 1 (SP1, p2), nucleocapsid protein (NC, p7), spacer peptide 2 (SP2, p1), P6, reverse transcriptase (RT), ribonuclease H (Rnase H), integrase (IN), HIV protease (PR), gp120, and gp41 protein of HIV virus comprise one or more proteolysis-targeting molecules and linkers.

In one embodiment of the present invention, by using reverse genetic technique, the inventors can prepare other subtypes or strains of influenza virus by replacing any gene of an existing influenza virus model with a gene of another subtype or strain, or by replacing an existing influenza virus model with another subtype or strain, so that the method can be applied to any subtypes or strains of influenza virus, and the prepared virus can replicate in a large number in cell strains that can selectively cut off the proteolysis-targeting molecule, and will be recognized and degraded by the ubiquitin-protease system in normal host cells. In addition, the method may be applied to other subtypes or strains, including H1N1, H1N2, H1N3, H1N8, H1N9, H2N2, H2N3, H2N8, H3N1, H3N2, H3N8, H4N2, H4N4, H4N6, H4N8, H5N1, H5N2, H5N3, H5N6, H5N8, H5N9, H6N1, H6N2, H6N4, H6N5, H6N6, H6N8, H7N1, H7N2, H7N3, H7N7, H7N8, H7N9, H8N4, H9N1, H9N2, H9N5, H9N8, H10N3, H10N4, H10N7, H10N8, H10N9, H11N2, H11N6, H11N9, H12N1, H12N3, H12N5, H13N6, H13N8, H14N5, H15N2, H15N8, H16N3, H17N10 and H18N11. In addition, multivalent influenza viruses, such as multivalent influenza viruses containing the surface antigen of H1N1, H3N2, or influenza B virus, can also be prepared using reverse genetic technique. More importantly, mutant strong virulent and multivalent viruses prepared possess a very high safety and efficacy.

The present invention also provides a nucleic acid molecule encoding the proteolysis-targeting virus.

The present invention also provides a nucleic acid vector expressing the proteolysis-targeting virus.

In another aspect, the present invention provides a method for preparing the proteolysis-targeting virus, comprising the steps of:
1) construction of cell line: constructing a cell line that can stably express a protease capable of selectively cleaving the linker of proteolysis-targeting virus;
   preferably, the cell line is a mammalian cell line;
   more preferably, the cell line is selected from CHO cells, Vero cells, MDCK.2 cells, HEK293T cells, MDCK cells, A549 cells, BHK cells, BHK-21/BRS cells, Sp2/0 cells, HEK293 cells, 293F cells, HeLa cells, TZM-bl cells, Sup-T1 cells, MRC-5 cells and VMK cells, LLC-MK2 cells, HCT-8 cells, Huh-7 cells, and Caco2 cells;
   still more preferably, the cell line is selected from HEK293T cell line or MDCK cell line;
   preferably, the cell line is optionally a ubiquitin-proteasome system-deficient cell line; more preferably, the cell line is a cell line with E3 ligase knockout or knockdown;
2) site selection: determining the type of proteolysis-targeting molecule to be introduced and the viral protein and site into which the proteolysis-targeting molecule and linker are introduced, through statistical analysis on the expression distribution of the ubiquitin-proteasome system in a host, and bioinformatic and protein structural prediction of the virus;
3) gene mutation: introducing a nucleotide sequence encoding the proteolysis-targeting molecule and linker into the selected site in the encoding gene of determined viral protein using a genetic engineering method;
4) construction of expression vector: operably linking the encoding nucleotide sequence of genetically mutated viral protein obtained in step 3) to a vector to obtain an expression vector;
   preferably, the expression vector is a plasmid;
5) cotransfecting the expression vector in step 4) and other expression vector for rescue of influenza virus in the cell line capable of selectively cleaving the linker of proteolysis-targeting virus constructed in step 1) using reverse genetic technology, to obtain the proteolysis-targeting virus;
   optionally, 6) replicating the proteolysis-targeting virus in the cell line obtained in step 1) to produce the proteolysis-targeting virus on a large scale;
   preferably, a proteasome inhibitor is added during the preparation of the virus;
      more preferably, the proteasome inhibitor is MG132, MG-341 or lactacystin;
   or
   the method comprising the steps of:
      (i) site selection: determining the type of proteolysis-targeting molecule to be introduced and the viral protein and site into which the proteolysis-targeting molecule and linker are introduced, through statistical analysis on the expression distribution of the ubiquitin-proteasome system in a host, and bioinformatic and protein structural prediction on the virus;
      (ii) gene mutation: introducing a nucleotide sequence encoding the proteolysis-targeting molecule and linker into the selected site in the encoding gene of determined viral protein using a genetic engineering method;
      (iii) construction of expression vector for mutated sequence: operably linking the encoding nucleotide sequence of genetically mutated viral protein obtained in step 3) to a vector to obtain an expression vector;
         preferably, the expression vector is a plasmid;
      (iv) constructing an overexpression vector of a protease capable of selectively cleaving the linker of the proteolysis-targeting virus;
         constructing a cell line that can stably express a protease capable of selectively cleaving the linker of the proteolysis-targeting virus;
      (v) cotransfecting the expression vector obtained in step (iii), other expression vectors required for virus rescue, and the expression vector obtained in step (iv) into host cells using reverse genetic technology, and culturing the successfully transfected host cells in a culture medium, to obtain the proteolysis-targeting virus.

The method according to the present invention further comprises step 7): detection: determining whether the proteolysis-targeting virus has been successfully modified by determining the replication capacity of the proteolysis-targeting virus obtained in step 5) in the cell line obtained in step 1) and in normal host cells without being modified, wherein the proteolysis-targeting virus that replicates in the cell line obtained in step 1), and has reduced or no replication capacity in normal host cells without being modified is a successfully modified proteolysis-targeting virus.
optionally, the method further comprises step 8):
using the successfully modified proteolysis-targeting virus vector, repeating steps 2)-5), so that the proteolysis-targeting molecule and linker are introduced into multiple viral proteins of the proteolysis-targeting virus, or multiple proteolysis-targeting molecules and linkers are introduced into any viral protein of the proteolysis-targeting virus;
optionally, determining whether the proteolysis-targeting virus has been successfully modified by determining the replication capacity of the obtained proteolysis-targeting virus in the cell line obtained in step 1) and in normal host cells without being modified, wherein the proteolysis-targeting virus that replicates in the cell line obtained in step 1), and has reduced or no replication capacity in normal host cells without being modified is a successfully modified proteolysis-targeting virus.

According to a specific embodiment of the present invention, provided is a method for preparation and large-scale production of a proteolysis-targeting influenza virus, comprising the steps of:
1) construction of cell line: stably transducing the tobacco etch virus protease TEVp into a mammalian cell line, and constructing the cell line that can stably express the tobacco etch virus protease TEVp;
   preferably, the mammalian cell line is HEK293T cell line and MDCK cell line;
   the stable cell lines constructed are HEK293T-TEVp and MDCK-TEVp; it should be noted that although in this embodiment, tobacco etch virus protease (TEVp) and its corresponding cleavable sequences are preferred as the objects of study, those skilled in the art should understand that the principles of technologies of the present invention can be extended to any other cleavable molecules (including proteins, polypeptides, nucleic acids, and chemical molecules);
2) site selection: through statistical analysis on the expression distribution of the ubiquitin-proteasome system in a host, and bioinformatic and protein structural prediction of influenza virus, predicting and analyzing the protein structure of influenza virus with a proteolysis-targeting molecule and linker, i.e., a sequence that can be cleaved by tobacco etch virus protease introduced into each protein of influenza virus, to determine the gene fragment and site into which the proteolysis-targeting molecule and linker introduced;
   preferably, one or more insertion sites are selected from the gene fragments encoding different proteins of influenza virus;
3) gene mutation: introducing a nucleotide sequence encoding the proteolysis-targeting molecule and linker into the selected site in the encoding gene of determined influenza viral protein using a genetic engineering method;
4) plasmid construction: operably linking the encoding nucleotide sequence of viral protein genetically mutated obtained in step 3) to a plasmid to obtain an encoding plasmid;
5) using reverse genetic technology, cotransfecting the plasmid in step 4) and other plasmid for rescue of influenza virus into the stable cells that stably express TEVp obtained in step 1) to obtain a proteolysis-targeting influenza virus;
   wherein the nucleotide sequence encoding the proteolysis-targeting molecule and the sequence to be cleaved by tobacco etch virus protease is introduced into the genome of the proteolysis-targeting influenza virus, thereby introducing the proteolysis-targeting molecule and the sequence to be cleaved by tobacco etch virus protease into the protein of corresponding influenza virus to obtain a proteolysis-targeting molecule modified influenza virus (PROTAC influenza virus);
   preferably, the method further comprises culturing the successfully transfected host cells in a DMEM medium containing 0.5% FBS and 2 µg/mL TPCK-trypsin;
   briefly, an expression vector of obtained mutated sequence, other plasmid required for virus rescue and a plasmid over-expressing TEVp are cotransfected into host cells, and the successfully transfected host cells are cultured in a DMEM medium containing 0.5% FBS and 2 µg/mL TPCK-trypsin;
   optionally, 6) producing the proteolysis-targeting influenza virus in a large-scale in a stable cell line that stably expresses TEVp;
   preferably, the supernatant is collected for infection of new MDCK-TEVp cells in a DMEM medium containing 0.5% FBS and 2 µg/mL TPCK-trypsin, after about 4 days of transfection or when the packaged virus has caused cytopathic alteration in all or more than 90% of the host cells in 4), and after 4 days of infection or when the infected virus has caused cytopathic alteration in all of the host cells in 4), the supernatant is collected to obtain it.

Preferably, the method further comprises step 7):
detection: determining whether the proteolysis-targeting influenza virus has been successfully modified by determining the dependence of proteolysis-targeting influenza virus obtained in step 5) on TEVp and the dependence of inactivation of its packaged product on the proteasome pathway;
specifically, the supernatant is centrifuged and passed through a 0.45 µm filter membrane to remove cell debris, and the packaged product is determined for TEVp dependence and the dependence of inactivation of packaged product on the proteasome pathway, and the mutant that maintains the TEVp dependence is retained and set as a successfully modified candidate. By TEVp dependence of packaged product, it is meant that the virus packaged using the described method can replicate and proliferate in cell lines with high TEVp expression, and has a reduced or defective replication capacity in normal cells that do not express TEVp.

Optionally, the method further comprises step 8):
using the successfully modified proteolysis-targeting influenza virus vector, repeating steps 2)-5), so that the proteolysis-targeting molecule and linker are introduced into each of multiple viral proteins of the proteolysis-targeting influenza virus, or multiple proteolysis-targeting molecules and linkers are introduced into any viral protein of the proteolysis-targeting influenza virus;
preferably, determining whether the proteolysis-targeting virus has been successfully modified by determining the dependence of obtained proteolysis-targeting influenza virus on TEVp and the dependence of inactivation of its packaged product on the proteasome pathway, and retaining the proteolysis-targeting virus that still maintains the dependence on TEVp after a long-term passage as a successfully modified candidate.

Optionally, the method further comprises:
step 9) selecting the successfully modified candidate and purifying the product;
step 10) performing a safety or immunogenicity detection on the proteolysis-targeting influenza virus in step 9), wherein compared with the wild type virus, the safer influenza virus is a successfully modified influenza virus.

In a specific embodiment of the present invention, PROTAC influenza virus is obtained by inserting a proteolysis-targeting influenza virus gene into a vector (e.g. pHH21 plasmid), and transfecting the vector together with other vector required for rescue of influenza virus into a stable cell line that can selectively cut off the proteolysis-targeting molecule, preferably HEK293T-TEVp and MDCK-TEVp. The virus is amplified and prepared in a large quantity in a stable cell line, preferably HEK293T-TEVp and/or MDCK-TEVp, that can selectively cut off the proteolysis-targeting molecule.

In one embodiment of the present invention, in order to improve the output efficiency of influenza virus as well as implement industrial production in the future, the inventors established stable cell lines (preferably HEK293T-TEVp and MDCK-TEVp) that can stably express the tobacco etch virus protease TEVp. These mammalian stable cell lines also overcome the disadvantage that the conventional use of chicken embryos for propagating the virus is prone to adverse reactions, such as allergy in human. To further improve the output efficiency, an appropriate amount of proteasome inhibitor can be added into a virus medium to inhibit the degradation of viral protein by the ubiquitin-proteasome system. Alternatively, ubiquitin-proteasome system deficient cell lines, such as cell lines with E3 ubiquitin ligase knockout or knockdown, can be used.

The present invention provides a mutant influenza virus, i.e., PROTAC virus, which can be prepared from mammalian cells by introducing a proteolysis-targeting molecule that can be conditionally cleaved into the genome and the corresponding protein, respectively. Preferably, the virus is H1N1, H5N1, H7N9, H3N2 or B influenza virus.

The above viruses can also be prepared from a stable mammalian cell line stably expressing TEVp (e.g. 293T cells, MDCK cells, CHO cells, Vero cells, and etc.). To further improve the output efficiency, an appropriate amount of proteasome inhibitor can be added to a virus medium to inhibit the degradation of viral protein by the ubiquitin-proteasome system. Alternatively, the ubiquitin-proteasome system deficient cell lines, such as cell lines with E3 ubiquitin ligase knockout or knockdown, can be used. The prepared mutant influenza viruses have a good safety and immunological activity.

In order to establish mammalian cell lines stably expressing TEVp, the inventors constructed a lentiviral overexpression vector with puromycin resistance, which carries the expression gene of TEVp, and transduces HEK293T cells and MDCK cells by the virus respectively, and stable cell strains stably expressing TEVp are obtained through puromycin screening. These stable cell strains were monoclonalized, and subjected to monoclonal culture, and the stable cell strains with the highest TEVp expression amount were sorted out using Western blotting and RT-qPCR, which are the final stable cell lines, HEK293T cells and MDCK cells.

The purified mutant PROTAC influenza virus can be obtained by a hollow fiber column and a gel chromatography method or sucrose gradient density centrifugation method. In vitro and in vivo experiments preliminarily demonstrate that PROTAC influenza viruses have an excellent safety and genetic stability, and better immune effect compared with inactivated virus.

The present invention also provides a method for preparing an attenuated live virus, replication-incompetent live virus, or replication-controllable live virus, and for preparing a relevant vaccine and medicament for preventing and treating viral infections, comprising a step of using the proteolysis-targeting virus or a step of preparing a proteolysis-targeting virus using the method.

The present invention also provides a vaccine or pharmaceutical composition, comprising the proteolysis-targeting virus.

Preferably, the vaccine is an attenuated live vaccine, replication-incompetent live vaccine or replication-controllable live vaccine.

The present invention also provides a system for preparing the proteolysis-targeting virus, comprising: a cell line that stably expresses a protease capable of selectively cleaving a linker of proteolysis-targeting virus. Preferably, the cell line is a cell line that stably expresses the tobacco etch virus protease TEVp. More preferably, the cell line is HEK293T cell line or MDCK cell line that stably expresses the tobacco etch virus protease TEVp. In order to further improve the output efficiency, an appropriate amount of proteasome inhibitor can be added into a virus medium to inhibit the degradation of viral protein by the ubiquitin-proteasome system. Alternatively, the ubiquitin-proteasome system deficient cell lines, such as a cell line with E3 ubiquitin ligase knockout or knockdown, can be used.

Further preferably, the system further comprises a nucleic acid vector expressing the proteolysis-targeting virus.

In yet another aspect, the present invention provides use of the proteolysis-targeting virus of the present invention in the preparation of an attenuated live vaccine, replication-incompetent live vaccine, or replication-controllable live vaccine, and in the preparation of a medicament for preventing and treating viral infections.

The present invention also provides a vaccine, containing PROTAC influenza virus, PROTAC novel coronavirus or PROTAC HIV virus.

Preferably, the vaccine comprises an adjuvant and other excipient.

The present invention also provides a pharmaceutical composition comprising the PROTAC influenza virus, PROTAC novel coronavirus or PROTAC HIV virus, and a pharmaceutically acceptable carrier, diluent or excipient.

The present invention also provides use of PROTAC influenza virus, PROTAC novel coronavirus or PROTAC HIV virus in the preparation of an attenuated live vaccine, replication-incompetent live vaccine, or replication-controllable live vaccine, and in the preparation of a medicament for preventing and treating viral infections.

The invention also further provides a lentiviral vector carrying the gene of tobacco etch virus protease (TEVp), the sequence of which is shown below. The vector is packaged into the virus and then transduced into cells. The cells are screened using puromycin. Tobacco etch virus protease (TEVp) can be integrated into host cells. The amino acid sequence of TEVp is as described in SEQ ID NO: 150:
SEQ ID NO: 150:

MGESLFKGPRDYNPISSTICHLTNESDGHTTSLYGIGFGPFIITNKHLFRRN
NGTLLVQSLHGVFKVKNTTTLQQHLIDGRDMIIIRMPKDFPPFPQKLKFREPQR
EERICLVTTNFQTKSMSSMVSDTSCTFPSSDGIFWKHWIQTKDGQCGSPLVSTR
DGFIVGIHSASNFTNTNNYFTSVPKNFMELLTNQEAQQWVSGWRLNADSVLW
GGHKVFMVKPEEPFQPVKEATQLMN

The present invention also provides a stable mammalian cell line that stably expresses TEVp, HEK293T-TEVp, or MDCK-TEVp.

The stable cell lines MDCK-TEVp and HEK293T-TEVp are obtained by lentiviral transduction, and carry the gene of tobacco etch virus protease (TEVp). Using this stable cell line, a proteolysis-targeting molecule with a TEVp cleavable sequence as a linker can be introduced into a specific site of any target protein of the virus, enabling a large-scale preparation of PROTAC virus.

The compositions, pharmaceutical compositions and vaccines described above can be prepared using conventional techniques in the art based on the preparation of PROTAC influenza virus modified by site-directed mutation according to the present invention, and used to prevent or treat influenza virus infections, including humans and animal influenza virus infections.

In a further aspect, the present invention provides a live influenza virus vaccine with site-directed mutation and site-directed modification, replication of which is controllable, wherein the site-directed mutation and site-directed modification are introduction of a peptide fragment, amino acid sequence, or other molecule that can cause protein degradation (hydrolysis) into any protein of influenza virus, and the introduced peptide fragment, amino acid sequence, or other molecule that can cause protein degradation (hydrolysis) is selectively cut off, inhibited, or inactivated during the preparation of the virus. The present invention also relates to the preparation of an influenza virus in which a peptide fragment, amino acid sequence, or other molecule that can cause protein degradation (hydrolysis) into multiple gene fragments by combining the sites modified by site-directed mutation.

The present invention also provides a method for site-directed mutation and modification of an influenza virus, comprising introducing any molecule (including a polypeptide, amino acid sequence, protein, or other molecule) that can cause protein degradation (hydrolysis), i.e., a proteolysis-targeting molecule, into any gene (protein) of influenza virus via zero, one, or more selectively cleavable linkers (including a polypeptide, amino acid sequence, protein, or other molecule). The influenza virus protein modified by mutation can be degraded (hydrolyzed) in normal host (cell), resulting in reduction or even complete loss of the replication capacity (activity) of the virus.

The present invention also provides a method for selectively inhibiting degradation (hydrolysis) of viral protein, comprising selectively inhibiting or inactivating a molecule that can cause degradation (hydrolysis) of the protein, and selectively cleaving a linker between the protein degradation molecule and the viral protein to ensure a high production efficiency of the virus during the preparation process. To further improve the production efficiency of PROTAC virus, an appropriate amount of proteasome inhibitor (e.g. MG132, MG-341, lactacystin, or etc.) can also be added for inhibiting the protein degradation mediated by the proteasome during the preparation of PROTAC virus, or ubiquitin-proteasome system deficient cells, such as a cell line with corresponding E3 ligase knockout or knockdown, are used for the preparation of PROTAC virus.

The present invention further provides use of influenza virus modified by site-directed mutation or combined at multiple sites, such as use as a live influenza virus vaccine, attenuated vaccine, replication-incompetent vaccine, safety model for strong virulent influenza virus, or etc. The techniques in the present invention can be used widely in applications of the modification and preparation of any virus, and/or live vaccine, attenuated vaccine, inactivated vaccine, safety model for strong virulence, and etc.

Compared with the prior art, the present invention has the following advantages:
(1) Versatility: all viruses have proteins, and the replication of virus can be controlled by introducing "a cleavable proteolysis-targeting molecule" into the viral protein to be degraded. Therefore, this technology can be used for the preparation of vaccines for all viruses.
(2) There are thousands of proteolysis-targeting molecules available, all of which can be exploited for the preparation of such viral vaccines. So there are rich alternative reserve resources.
3) Simple operation: only simple techniques of virus vector construction and virus preparation are required, and producers can prepare such vaccines even without much knowledge about virus biology.
4) Experiments have demonstrated that this technology can be used to design vaccines with different levels of inactivation, and thus has safe controllability.
5) Experiments have demonstrated that such vaccines have a good immunogenicity.

### Brief Description of the Drawings

Hereinafter, the embodiments of the present invention are illustrated in detail in conjunction with the accompanying drawings, wherein:
FIG. 1 is a map of TEVp overexpression lentiviral vector.
FIG. 2A is an electrophoretogram of RT-PCR identification of potential HEK293T-TEVp cells (the cells indicated by the arrows are those with a relatively high mRNA expression level of the target gene TEVp); FIG. 2B is an electrophoretogram of RT-PCR identification of potential MDCK-TEVp cells (O (old) represents the first batch of MDCK cells, and N (new) represents the second batch of MDCK cells; and the cells indicated by the arrows are those with a relatively high mRNA expression level of the target gene TEVp).
FIG. 3 are graphs of protein expression assay of the target protein TEVp and virus packaging efficiency for HEK293T-TEVp and MDCK-TEVp cell lines prepared according to the method of the present invention.
FIG. 4 is a histogram of replication titers of proteolysis-targeting influenza viruses (PROTAC influenza viruses) prepared according to the method of the present invention 2 days after the infection in an MDCK-TEVp cell line and normal cell line.
FIG. 5 is a histogram of replication titers of proteolysis-targeting influenza viruses (PROTAC influenza viruses) comprising multiple proteolysis molecules prepared according to the method of the present invention after the infection in an MDCK-TEVp cell line and normal cell line.
FIG. 6 are histograms comparing the replication capacities of proteolysis-targeting influenza viruses (PROTAC influenza viruses) prepared according to the method of the present invention in an MDCK-TEVp cell and normal cell line.
FIG. 7 shows an investigation of genetic stability of proteolysis-targeting influenza viruses (PROTAC influenza viruses) prepared according to the method of the present invention, demonstrating that the prepared PROTAC influenza viruses remain stable after multiple passages.
FIG. 8 is a graph evaluating that the PROTAC influenza viruses according to the present invention can be degraded by the proteasome in normal cells using western blot assay.
FIG. 9 is a graph of immunofluorescence detection for evaluating the replication of PROTAC influenza viruses according to the present invention in an MDCK-TEVp cell line and normal cells using immunofluorescence assay.
FIG. 10 shows the immunogenicity and protection results for the PROTAC viruses according to the present invention at an animal level.
FIG. 11 provides a flow chart of the preparation method for preparing a proteolysis-targeting virus according to the method of the present invention.

### Best Modes for Carrying Out the Invention

In order to better understand the invention, the inventors illustrate and set forth specific experiments with examples, wherein the examples are used for illustrative purposes only and do not limit the protection scope of the present invention. Any variants or embodiments equivalent to the invention are included in the present invention.

### Example 1 Establishment of mammalian stable cell lines HEK293-TEVp and MDCK-TEVp that can stably express TEVp

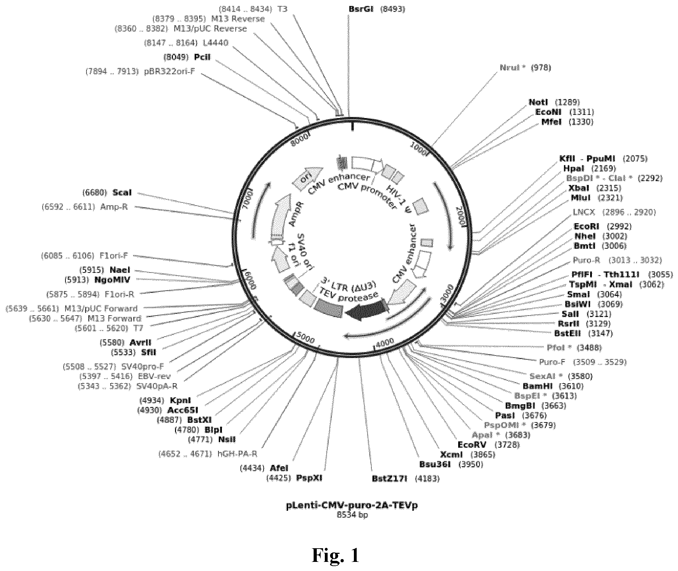

The mammalian cell lines stably expressing TEVp were completed with the assistance of Beijing CorreGene Biotechnology Co. Ltd. through a delegate.
(1) Construction of TEVp overexpression lentiviral vector:
   a map of TEVp overexpression lentiviral vector is shown in Figure 1, and the lentiviral vector has puromycin resistance.
(2) Lentiviral packaging, purification and quantification
   (i) cell inoculation on day 0: 293T was inoculated into 10 cm/15 cm culture dishes (the inoculation number was determined by the desired amount of virus, 1 × 10^8/15 cm dish), the inoculation density was controlled, and the cells grew to a confluence of 80% the next day;
   (ii) plasmid transfection on day 1: 293T cells were cotransfected using the TEVp overexpression lentiviral vector described in (1), psPAX2 plasmid (which is available from Addgene) and pVSVG plasmid (which is available from Addgene);
   (iii) medium replacement on day 2: the medium was replaced after 18 hours of transfection (the transfection system is toxic), the medium was completely suck off, and 20mL of fresh complete medium was carefully added (15cm culture dish);
   (iv) viral supernatant pretreatment: after collecting the viral supernatant on days 3, 4 and 5, the culture supernatant was transferred into a 50 mL centrifuge tube and subjected to centrifugation at the highest speed for 10min; the viral supernatant was filtered using a 0.45 µm filter and collected directly using a sterilized 250 mL centrifuge bottle;
   (v) virus purification by high-speed centrifugation: by using a 20 mL syringe, 10% sucrose solution was slowly injected into the bottom of centrifuge bottle, a volume ratio of 4:1 (4 parts of virus supernatant, 1 part of sucrose solution) was maintained, and centrifugation was performed at 4°C, 14000 rpm for 2 hours;
   (vi) virus resuspension: the supernatant was discarded and sucked off, and 100 ul-1000 ul PBS was added and mixed uniformly by sucking and blowing;
   (vii) virus supernatant was subpackaged and cryopreserved at -80 °C; about 30 ul of the remaining was cryopreserved separately at the time of subpackage for titer determination;
   (viii) chemiluminescence assay (CMIA) was used to determine the lentivirus titer, and the titer of pLenti-CMV-puro-2A-TEVp-8534bp was determined as 5.12E+7TU/mL, in a volume of 1000 ul with a total amount of 5.12 E+7TU, which meeted the transduction requirements.
(3) Construction of stable monoclonal cell line
   (i) cell inoculation at Day 0: HEK293T or MDCK was inoculated into a 12/6-well plate culture dish (the inoculation number was determined by the desired amount of virus, 1 × 10^6/6-well plate culture dish), the inoculation density was controlled, and the cells grew to a confluence of 20-30% the next day;
   (ii) virus thawing on day 1: the virus was taken from -80°C refrigerator and thawed on ice; the cells were infected using an appropriate moi (MOI=10);
   (iii) virus transduction on day 2: the cells were infected using an appropriate moi for 24 hours and then 2 µg/mL puro resistance medium was replaced for resistance screening;
   (iv) continuous resistance screening was performed for about 7 days (blank controls were all killed);
   (v) the surviving cells were digested and counted, and 100/200 cells were distributed equally into a 96-well plate (on average, 1-2 cells per well);
   (vi) clonal proliferation was visible in 3-5 days, the monoclonal cells contained the wells were selected and subjected to proliferation, and step-by-step expanded to 24-well plate and 12-well plate;
   (vii) monoclone identification:
      identification primers
      TEVp-F:
         SEQ ID NO: 151 TCATTACAAACAAGCACTTG
      TEVp-R:
         SEQ ID NO: 152 TAGGCATGCGAATAATTATC
      Fragment size: 144 bp
      293t-GAPDH-eF:
         SEQ ID NO: 153 CCACATCGCTCAGACACCAT
      293t-GAPDH-eR:
         SEQ ID NO: 154 GGCAACAATATCCACTTTACCAGAGT
      Fragment size: 114 bp

The preferred monoclonal cells were subjected to digestion, RNA extraction and RT-PCR identification to verify whether the cells integrated and expressed TEVp. The amplification conditions are shown in Table 1 below:

**Table 1 Amplification conditions**

| 1 cycle | 40 cycles | | | 1 cycle | 1 cycle |
|---|---|---|---|---|---|
| 94°C | 94°C | 55°C | 72°C | 72°C | 25°C |
| 5 min | 15 s | 15 s | 15 s | 5 min | ∞ |

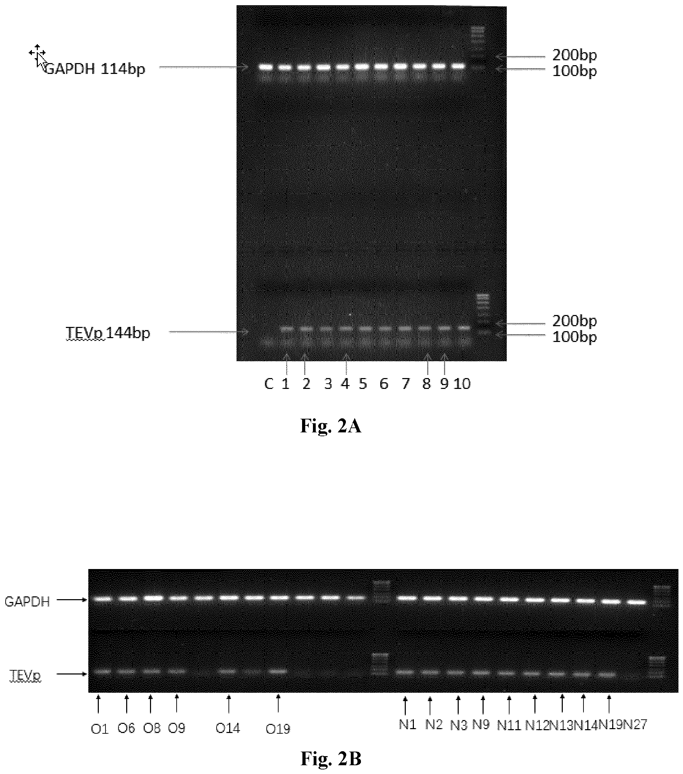

The results are shown in Figure 2. Figure 2A is an electrophoretogram of RT-PCR identification for potential HEK293T-TEVp cells (the cells indicated by the arrows are those with a relatively high mRNA expression level of the target gene TEVp). The verification work was completed with the assistance of Beijing CorreGene Biotechnology Co. Ltd. through a delegation. FIG. 2B is an electrophoretogram of RT-PCR identification of potential MDCK-TEVp cells (O (old) represents the first batch of MDCK cells, and N (new) represents the second batch of MDCK cells; and the cells indicated by the arrows are those with a relatively high mRNA expression level of the target gene TEVp). The verification work was completed with the assistance of Beijing CorreGene Biotechnology Co. Ltd. through a delegation.
(viii) amplification culture of preferred monoclonal cell lines
preferably, the cell lines with a high TEVp expression efficiency were expanded and cryopreserved for use, which were designated as HEK293T-TEVp and MDCK-TEVp, respectively.

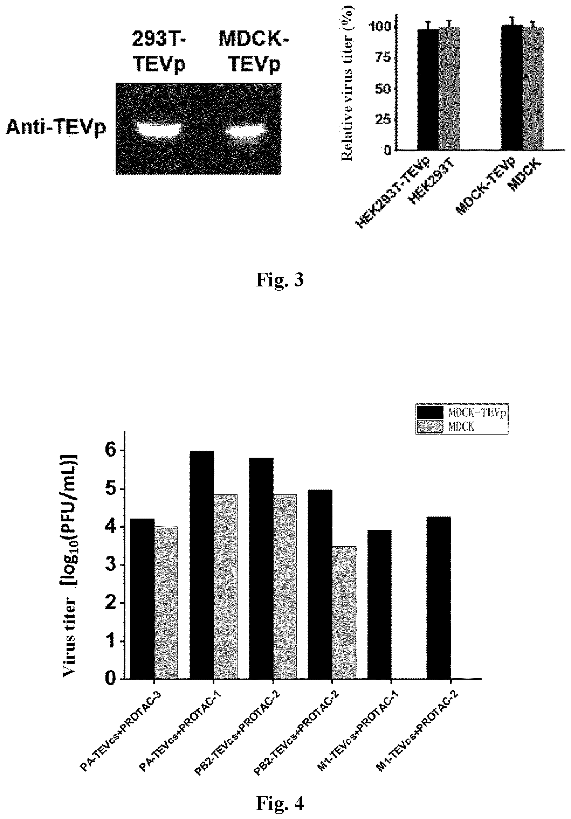

The results are shown in Figure 3, western blot assays were performed on HEK293T-TEVp and MDCK-TEVp cell lines using an anti-TEVp antibody. The results showed that the tobacco etch virus protease TEVp could be efficiently and stably expressed in the HEK293T-TEVp and MDCK-TEVp stable cell lines. Furthermore, the modification of cells did not cause changes of virus production efficiency, indicating the compatibility of TEVp in the cells.

### Example 2 Construction of gene vector for influenza virus WSN comprising cleavable proteolysis-targeting molecule

(1) Acquisition of plasmids for rescuing wild type influenza virus WSN:
According to the gene sequences of influenza virus A/WSN/1933 as published by pubmed
https://www.ncbi.nlm.nih.gov/nuccore/?term=WSN+PB2;
https://www.ncbi.nlm.nih.gov/nuccore/?term=WSN+PB1;
https://www.ncbi.nlm.nih.gov/nuccore/?term=WSN+PA;
https://www.ncbi.nlm.nih.gov/nuccore/?term=WSN+HA;
https://www.ncbi.nlm.nih.gov/nuccore/?term=WSN+NA;
https://www.ncbi.nlm.nih.gov/nuccore/?term=WSN+NS,

various gene fragments of this influenza virus gene were obtained by total gene synthesis. Then, they were linked to pHH21, pCDNA 3 (neo), and pcAAGGS/MCS vectors (obtained from Beijing Zhongke Yubo Biotechnology Co., Ltd.) respectively, to obtain the plasmids for rescuing wild type influenza virus WSN. The nomenclature and composition of obtained plasmids are shown in Table 2 below.

**Table 2**

| Abbreviation | Name of plasmid | Key gene | Restriction enzyme site | Structure of constructed plasmid |
|---|---|---|---|---|
| Ben1 | PHH21 | PB2 | BsmBI | pPolI-WSN-PB2 |
| Ben2 | PHH21 | PB1 | BsmBI | pPolI-WSN-PB 1 |
| Ben3 | PHH21 | PA | BsmBI | pPolI-WSN-PA |
| Ben4 | PHH21 | HA | BsmBI | pPolI-WSN-HA |
| Ben5 | PHH21 | NP | BsmBI | pPolI-WSN-NP |
| Ben6 | PHH21 | NA | BsmBI | pPolI-WSN-NA |
| Ben7 | PHH21 | M | BsmBI | pPolI-WSN-M |
| Ben8 | PHH21 | NS | BsmBI | pPolI-WSN-NS |
| Ben9 | pcDNA3(neo) | PB2 | EcoRI | pcDNA3(neo)-PB2 |
| Ben10 | pcDNA3(neo) | PB1 | EcoRI | pcDNA3(neo)-PB1 |
| Ben11 | pcDNA3(neo) | PA | EcoRI | pcDNA3(neo)-PA |
| Ben13 | pcAGGS/MCS | NP | EcoRI | pcAGGS/MCS-NP |

(2) Construction of viral vectors having introduced cleavable proteolysis-targeting molecule

The inventors introduced a gene sequence of proteolysis-targeting molecule that can be cleaved by TEVp into the C-terminus of gene coding region corresponding to each viral protein (PA, PB2, PB1, NP, HA, NA, M1, M2, NS1, and NEP) of the influenza virus WSN before the termination codon respectively, and constructed the following viral vectors.

Specifically, the gene sequences of proteolysis-targeting molecules that were introduced to any protein of the virus and can be cut off by TEVp, and the amino acid sequences expressed by them were as follows, but not limited to the following sequences. The gene sequences corresponding to the amino acid sequences used were optimized for humanization and inserted into the C-terminus of the encoding region of the target protein gene before the termination codon. This work was completed with the assistance of Beijing Tsingke Biotechnology Co., Ltd. through a delegation, and the successful construction of mutant was verificated by sequencing, wherein:
(1) the sequence as shown in SEQ ID NO: 138 was introduced into the C-terminus of PA protein of the influenza virus, i.e., the nucleotide sequence as shown in SEQ ID NO: 155 was introduced into the C-terminus of its PA protein gene encoding region before the termination codon, and the resultant sequence was named as PA-TEVcs+PROTAC-1 (or named as PA-PTD1);
   SEQ ID NO: 138 GSGGENLYFQGGSGALAPYIP;
   SEQ ID NO: 155 
GGTTCTGGTGGTGAGAAT CTGTAC TTC CAA GGTGGATCTGGAGCA
TTG GCC CCC TAC ATTCCA;
2) similarly, the sequence as shown in SEQ ID NO: 138 was introduced into the C-terminus of M1 protein of the influenza virus, i.e., the nucleotide sequence as shown in SEQ ID NO: 155 was introduced into the C-terminus of its PA protein gene encoding region before the termination codon, and the resultant sequence was named as M1-TEVcs+PROTAC-1 (or named as M1-PTD1);
(3) the sequence as shown in SEQ ID NO: 139 was introduced into the C-terminus of PB1 protein of the influenza virus, i.e., the nucleotide sequence as shown in SEQ ID NO: 156 was introduced into the C-terminus of its PB1 protein gene encoding region before the termination codon, and the resultant sequence was labeled as PB1-TEVcs+PROTAC-2 (or named as PB1-PTD2):
   SEQ ID NO: 139 GSGGENLYFQGGSGDRHDSGLDSM
   SEQ ID NO: 156 GGTTCTGGTGGTGAG AAT CTGTAC TTC CAA GGTGGATCTGGAGAT CGC CAC GAT TCA GGG CTC GAT TCC ATG
4) the sequence as shown in SEQ ID NO: 139 was introduced into the C-terminus of PB2 protein of the influenza virus, i.e., the nucleotide sequence as shown in SEQ ID NO: 156 was introduced into the C-terminus of its PB2 protein gene encoding region before the termination codon, and the resultant sequence was labeled as PB2-TEVcs+PROTAC-2 (or named as PB2-PTD2);
5) the sequence as shown in SEQ ID NO: 139 was introduced into the C-terminus of M1 protein of the influenza virus, i.e., the nucleotide sequence as shown in SEQ ID NO: 156 was introduced into the C-terminus of its M1 protein gene encoding region before the termination codon, and the resultant sequence was labeled as M1-TEVcs+PROTAC-2 (or named as M1-PTD2);
6) the sequence as shown in SEQ ID NO: 140 was introduced into the C-terminus of PA protein of the influenza virus, i.e., the nucleotide sequence as shown in SEQ ID NO: 157 was introduced into the C-terminus of its PA protein gene encoding region before the termination codon, and the resultant sequence was named as PA-TEVcs+PROTAC-3 (or named as PA-PTD3);
   SEQ ID NO: 140: 
GSGGENLYFQGGGGSSHGFPPEVEEQDDGTLPMSCAQESGMDRHPAAC
ASARINV;
 SEQ ID NO: 157 
GGTTCTGGTGGTGAGAATCTGTACTTCCAAGGTGGAGGAGGATCCAG
CCATGGCTTCCCGCCGGAGGTGGAGGAGCAGGATGATGGCACGCTGCCCA
TGTCTTGTGCCCAGGAGAGCGGGATGGACCGTCACCCTGCAGCCTGTGCTT
CTGCTAGGATCAATGTG
(7) PA-TEVcs+PROTAC-1 (PA-PTD1) and PB1-TFVcs+PROTAC-2 (PB1-PTD2) were combined to construct a virus carrying a cleavable proteolysis molecule in PA and PB1, respectively.

Further, the inventors also constructed the following viral vectors:

**Table 3 Viral vectors**

| Viral vector naming | Introduced amino acids | Introduced nucleotides |
|---|---|---|
| PA-TEVcs+TEVcs +PROTAC-1 | SEQ ID NO: 141: | SEQ ID NO: 158 |
| | | GGTTCTGGTGGTGAGAATCTGTACTTCCAAGGTG GATCTGGAGAAAACCTCTATTTTCAGTCAGGTAG TGGTGCATTGGCCCCCTACATTCCA |
| PB2/PB1-TEVcs+ TEVcs+PROTAC -2 | SEQ ID NO: 142 | SEQ ID NO: 159 |
| | | GGTTCTGGTGGTGAGAATCTGTACTTCCAAGGTG GATCTGGAGAAAACCTCTATTTTCAGTCAGGTAG TGGTGATCGCCACGATTCAGGGCTCGATTCCATG |
| PA-TEVcs+ TEVcs+PROTAC -3 | SEQ ID NO: 143 | SEQ ID NO: 160 |
| | | GGTTCTGGTGGTGAGAATCTGTACTTCCAAGGTG GAGGAGGATCCGAAAACCTCTATTTTCAGTCAGG TAGTGGTAGCCATGGCTTCCCGCCGGAGGTGGAG GAGCAGGATGATGGCACGCTGCCCATGTCTTGTG CCCAGGAGAGCGGGATGGACCGTCACCCTGCAG CCTGTGCTTCTGCTAGGATCAATGTG |
| PA-TEVcs+ PROTAC-1+ PROTAC-1 (or named as PA-PTD4) | SEQ ID NO: 144 | SEQ ID NO: 161 |
| | | GGTTCTGGTGGTGAGAATCTGTACTTCCAAGGTG GATCTGGAGCATTGGCCCCCTACATTCCAGGTAG TGGTGCCCTTGCACCATATATCCCC |
| PB2/PB1-TEVcs+ PROTAC-2+ PROTAC-1 (or named as PB2/PB1-PTD5) | SEQ ID NO: 145 | SEQ ID NO: 162 |
| | | GGTTCTGGTGGTGAGAATCTGTACTTCCAAGGTG GATCTGGAGATCGCCACGATTCAGGGCTCGATTC CATGGGATCTGGAGCATTGGCCCCCTACATTCCA |
| PA-TEVcs+ PROTAC-3+ PROTAC-1 | SEQ ID NO: 146 | SEQ ID NO: 163 |
| | | GGTTCTGGTGGTGAGAATCTGTACTTCCAAGGTG GAGGAGGATCCAGCCATGGCTTCCCGCCGGAGG |
| | | TGGAGGAGCAGGATGATGGCACGCTGCCCATGTC TTGTGCCCAGGAGAGCGGGATGGACCGTCACCCT GCAGCCTGTGCTTCTGCTAGGATCAATGTGGGAT CTGGAGCATTGGCCCCCTACATTCCA |
| PA-TEVcs+ TEVcs+PROTAC -1+PROTAC-1 (or named as PA-PTD8) | SEQ ID NO: 147 | SEQ ID NO: 164 |
| | | GGTTCTGGTGGTGAGAATCTGTACTTCCAAGGTG GATCTGGAGAAAACCTCTATTTTCAGTCAGGTAG TGGTGCATTGGCCCCCTACATTCCAGGTAGTGGT GCCCTTGCACCATATATCCCC |
| PB2/PB 1-TEVcs+ TEVcs+PROTAC -2+PROTAC-1 (or named as PB2/PB 1-PTD9) | SEQ ID NO: 148 | SEQ ID NO: 165 |
| | | GGTTCTGGTGGTGAGAATCTGTACTTCCAAGGTG GATCTGGAGAAAACCTCTATTTTCAGTCAGGTAG TGGTGATCGCCACGATTCAGGGCTCGATTCCATG GGTAGTGGTGCCCTTGCACCATATATCCCC |
| PA-TEVcs+ TEVcs+PROTAC -3+PROTAC-1 (or named as PA-PTD10) | SEQ ID NO: 149 | SEQ ID NO: 166 |
| | | GGTTCTGGTGGTGAGAATCTGTACTTCCAAGGTG GAGGAGGATCCGAAAACCTCTATTTTCAGTCAGG TAGTGGTAGCCATGGCTTCCCGCCGGAGGTGGAG GAGCAGGATGATGGCACGCTGCCCATGTCTTGTG CCCAGGAGAGCGGGATGGACCGTCACCCTGCAG CCTGTGCTTCTGCTAGGATCAATGTGGGTAGTGG TGCCCTTGCACCATATATCCCC |
| M1-TEVcs+ PROTAC-1+ PROTAC-2 (M1-PTD6) | SEQ ID NO: 167 GSGGENL YFQG GSGALAPYIPDR HDSGLDSM | SEQ ID NO: 169 |
| | | GGTTCTGGTGGTGAGAATCTGTACTTCCAAGGTG GATCTGGAGCATTGGCCCCCTACATTCCAGATCG CCACGATTCAGGGCTCGATTCCATG |
| PA-TEVcs+ PROTAC-3+ PROTAC-1 (PA-PTD7) | SEQ ID NO: 168 GSGGENL YFQG GGGSSHGFPPEV EEQDDGTLPMS CAQESGMDRHP AACASARINVGS GALAPYIP | SEQ ID NO: 170 |
| | | GGTTCTGGTGGTGAGAATCTGTACTTCCAAGGTG GAGGAGGATCCAGCCATGGCTTCCCGCCGGAGG TGGAGGAGCAGGATGATGGCACGCTGCCCATGT CTTGTGCCCAGGAGAGCGGGATGGACCGTCACCC TGCAGCCTGTGCTTCTGCTAGGATCAATGTGGGA TCTGGAGCATTGGCCCCCTACATTCCA |

**Table 3 (continued) Viral vectors**

| | | |
|---|---|---|
| PB2-TEVcs+PROTAC-1 (named as PB2-PTD1) | SEQ ID NO: 138 | SEQ ID NO: 155 |
| PB1-TEVcs+PROTAC-1 (named as PB1-PTD1) | SEQ ID NO: 138 | SEQ ID NO: 155 |
| HA-TEVcs+PROTAC-1 (named as HA-PTD1) | SEQ ID NO: 138 | SEQ ID NO: 155 |
| NP-TEVcs+PROTAC-1 (named as NP-PTD1) | SEQ ID NO: 138 | SEQ ID NO: 155 |
| NA-TEVcs+PROTAC-1 (named as NA-PTD1) | SEQ ID NO: 138 | SEQ ID NO: 155 |
| M2-TEVcs+PROTAC-1 (named as M2-PTD1) | SEQ ID NO: 138 | SEQ ID NO: 155 |
| NS-TEVcs+PROTAC-1 (named as NS-PTD1) | SEQ ID NO: 138 | SEQ ID NO: 155 |
| NEP-TEVcs+PROTAC-1 (named as NEP-PTD1) | SEQ ID NO: 138 | SEQ ID NO: 155 |
| PA-TEVcs+PROTAC-2 (or named as PA-PTD2) | SEQ ID NO: 139 | SEQ ID NO: 156 |
| HA-TEVcs+PROTAC-2 (or named as HA-PTD2) | SEQ ID NO: 139 | SEQ ID NO: 156 |
| NP-TEVcs+PROTAC-2 (or named as NP-PTD2) | SEQ ID NO: 139 | SEQ ID NO: 156 |
| NA-TEVcs+PROTAC-2 (or named as NA-PTD2) | SEQ ID NO: 139 | SEQ ID NO: 156 |
| M2-TEVcs+PROTAC-2 (or named as M2-PTD2) | SEQ ID NO: 139 | SEQ ID NO: 156 |
| NS-TEVcs+PROTAC-2 (or named as NS-PTD2) | SEQ ID NO: 139 | SEQ ID NO: 156 |
| NEP-TEVcs+PROTAC-2 (or named as NEP-PTD2) | SEQ ID NO: 139 | SEQ ID NO: 156 |
| M1-TEVcs+PROTAC-3 (or named as M1-PTD3) | SEQ ID NO: 140 | SEQ ID NO: 157 |
| PB2-TEVcs+PROTAC-3 (or named as PB2-PTD3) | SEQ ID NO: 140 | SEQ ID NO: 157 |
| PB 1-TEVcs+PROTAC-3 (or named as PB1-PTD3) | SEQ ID NO: 140 | SEQ ID NO: 157 |
| HA-TEVcs+PROTAC-3 (or named as HA-PTD3) | SEQ ID NO: 140 | SEQ ID NO: 157 |
| NP-TEVcs+PROTAC-3 (or named as NP-PTD3) | SEQ ID NO: 140 | SEQ ID NO: 157 |
| NA-TEVcs+PROTAC-3 (or named as NA-PTD3) | SEQ ID NO: 140 | SEQ ID NO: 157 |
| M2-TEVcs+PROTAC-3 (or named as M2-PTD3) | SEQ ID NO: 140 | SEQ ID NO: 157 |
| NS-TEVcs+PROTAC-3 (or named as NS-PTD3) | SEQ ID NO: 140 | SEQ ID NO: 157 |
| NEP-TEVcs+PROTAC-3 (or named NEP-PTD3) | SEQ ID NO: 140 | SEQ ID NO: 157 |
| M1-TEVcs+PROTAC-2+PROTAC-1 (or named as M1-PTD5) | SEQ ID NO: 145 | SEQ ID NO: 162 |

### Example 3: Rescue of PROTAC influenza viruses modified by site-directed mutation

12 plasmids used for rescue of influenza virus were cotransfected into a stable cell line according to a normal method for rescue of influenza virus, and the corresponding plasmids of these 12 plasmids were replaced with the plasmids modified by site-directed mutation in Example 2. For each well of six-well plate, 0.2 µg of each plasmid was added. After transfection, the cells were observed for cytopathic alteration and screened for insertion sites, proteolysis-targeting molecules, linkers cleavable by TEVp, and combinations thereof that could rescue the virus and were TEVp-dependent. The screened strains were named according to the protein and the introduced cleavable proteolysis-targeting molecule.

For illustration, after introducing the cleavable proteolysis-targeting molecule TEVcs+PROTAC-1 into Ben3 pPolI-WSN-PA plasmid, this plasmid and the other plasmid Ben1 pPolI-WSN-PB2, Ben2 pPolI-WSN-PB1, Ben4 pPolI-WSN-HA, Ben5 pPolI-WSN-NP, Ben6 pPolI-WSN-NA, Ben7 pPolI-WSN-M, Ben8 pPolI-WSN-NS, Ben9 pcDNA 3 (neo)-PB2, Ben10 pcDNA 3 (neo)-PB1, Ben11 pcDNA 3 (neo)-PA, or Ben13 pcAGGS/MCS-NP that rescues influenza virus were cotransfected into the stable cell line established in Example 1, thereby rescuing a mutant influenza virus with TEVCs+PROTAC-1 introduced into the PA gene fragment of influenza virus, which was named as PA-TEVcs+PROTAC-1 (or named as PA-PTD1). After the cleavable proteolysis-targeting molecule TEVcs+PROTAC-1 was introduced into the C-terminus of M1 protein encoding region in the Ben7 pPolI-WSN-M plasmid, the plasmid and the other plasmid Ben1 pPolI-WSN-PB2, Ben2 pPolI-WSN-PB1, Ben3 pPolI-WSN-PA, Ben4 pPolI-WSN-HA, Ben5 pPolI-WSN-NP, Ben6 pPolI-WSN-NA, Ben8 pPolI-WSN-NS, Ben9 pcDNA 3 (neo)-PB2, Ben10 pcDNA 3 (neo)-PB1, Ben11 pcDNA 3 (neo)-PA, or Ben13 pcAGGS/MCS-NP that rescues influenza virus were cotransfected into the stable cell line established in Example 1, thereby rescuing a mutant influenza virus with TEVCs+PROTAC-1 introduced into the M1 gene fragment of influenza virus, which was named as M1-TEVcs+PROTAC-1 (or named as M1-PTD1).

Following the same approach, mutant influenza viruses with cleavable proteolysis-targeting molecule introduced into other sites can be obtained and named according to the same rules.

All the constructed PROTAC virus vectors were investigated according to the criterion whether they could cause cytopathic alteration: if they could cause cytopathic alteration in HEK293T-TEVp and/or MDCK-TEVp, it demonstrated that the rescue of PROTAC virus was successful; if they fail to cause cytopathic alteration in HEK293T-TEVp and/or MDCK-TEVp, it demonstrated that the rescue of PROTAC virus failed. The examples of rescue of partial mutant influenza viruses can be seen in Table 4 and Figure 4. A portion of PROTAC influenza strains that can cause cytopathic alteration in MDCK-TEVp cells are summarized in Table 4. Figure 4 shows the detected virus titers of cell supernatants the next day after infection after a portion of strains were inoculated into MDCK-TEVp cells and normal MDCK cells (MOI=0.01). The results indicate that selectively cleavable proteolysis-targeting molecule can be introduced into each of eight gene fragments of influenza virus, wherein the PROTAC influenza virus can be rescued in terms of PA, PB2, PB1, M1, and etc.

**Table 4 Partial strains of PROTAC influenza virus causing cytopathic alteration in MDCK-TEVp cells**

| Name of strain | Second name of strain | Whether it causes cytopathic alteration? |
|---|---|---|
| PA-TEVcs+PROTAC-1 | PA-PTD1 | yes |
| M1-TEVcs+PROTAC-1 | M1-PTD1 | yes |
| PB2-TEVcs+PROTAC-2 | PB2-PTD2 | yes |
| PB 1 -TEVcs+PROTAC-2 | PB1-PTD2 | yes |
| M1-TEVcs+PROTAC-2 | M1-PTD2 | yes |
| PA-TEVcs+PROTAC-3 | PA-PTD3 | yes |
| PA-TEVcs+PROTAC-1+PROTAC-1 | PA-PTD4 | yes |
| M1-TEVcs+PROTAC-2+PROTAC-1 | M1-PTD5 | yes |
| M1-TEVcs+PROTAC-1+PROTAC-2 | M1-PTD6 | yes |
| PA-TEVcs+TEVcs+PROTAC-1 +PROTAC-1 | PA-PTD8 | yes |
| PA-TEVcs+PROTAC-1 + PB 1 -TEVcs+PROTAC-2 | PTOTAC-V1 | yes |

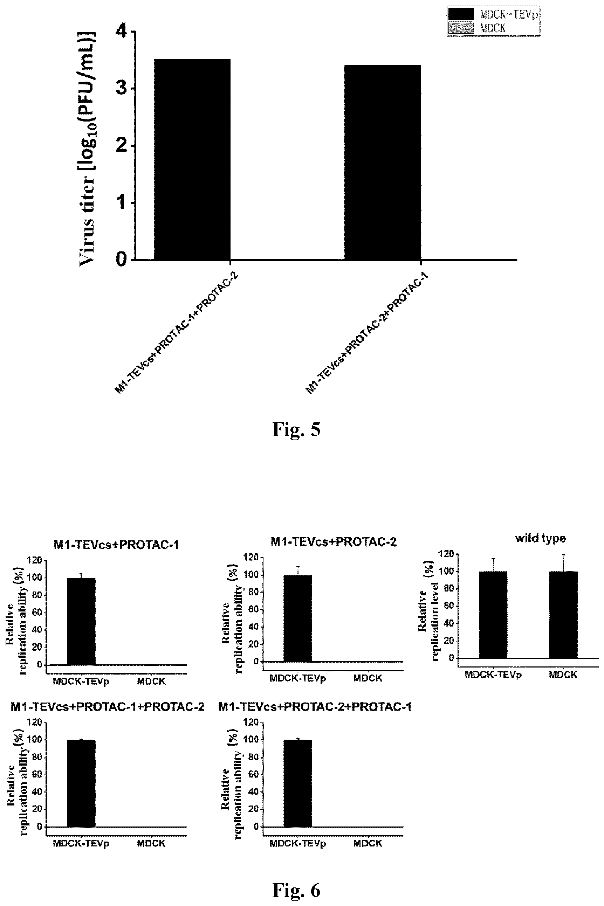

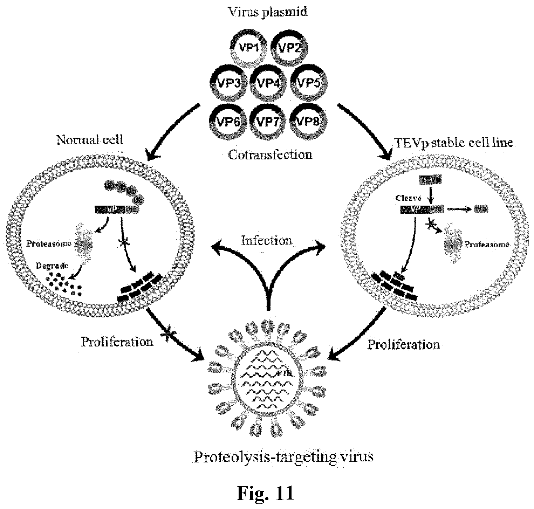

From the above-mentioned mutation sites and cleavable proteolysis-targeting molecules, the inventors selected the mutational modifications that were highly efficient in rescuing influenza virus, genetically stable, and resulted in a significantly reduction or even complete loss of eventual replicate capacity in normal host cells, and combined them to prepare influenza viruses comorising multiple proteolysis-targeting molecules, further selected preferred strains therefrom, and named as M1-TEVcs+PROTAC-1+PROTAC-2 (or M1-PTD6), M1-TEVcs+PROTAC-2 +PROTAC-1 (or M1-PTD5), and PROTAC-V1 (Table 4 and Figure 5). M1-TEVcs+ PROTAC-1+PROTAC-2 is a PROTAC strain comprising two proteolysis-targeting molecules, which was prepared by introducing PROTAC-1 and PROTAC-2 in tandem into the C-terminus of M1 protein of the virus. M1-TEVcs+PROTAC-2+PROTAC-1 is a PROTAC strain comprising two proteolysis-targeting molecules, which was prepared by introducing PROTAC-2 and PROTAC-1 in tandem into the C-terminus of M1 protein of the virus. PROTAC-VI is a PROTAC strain comprising two proteolysis-targeting molecules, which was prepared by introducing TEVcs-PROTAC-1 into the C-terminus of PA protein of the virus and TEVcs-PROTAC-2 into the C-terminus of PB1 protein of the virus (Table 4). Further, Fig. 11 provides a flow chart of the preparation of proteolysis-targeting virus according to the method of the present invention.

### Example 4 Expression and purification of influenza viruses with site-directed mutation

### 1) Rescue of PROTAC viruses comprising a cleavable proteolysis-targeting molecule

The packaging plasmids of mutant influenza virus obtained in the rescue of influenza virus modified by site-directed mutation in the step of Example 3 were cotransfected into the stable cell line in Example 1, the medium was replaced with a new medium containing 1% FBS and 2 µg/mL of TPCK-trypsin after 6 hours, and normal cells were used as a control. The positive control used for this rescue experiment was wild type influenza virus WSN, and the conditions were the same as those for the rescue of mutant influenza virus, except that the plasmids for rescue of the virus were different. After the transfection was completed, the status of the cells was observed daily, and cytopathic alteration appeared in the TEVp stable cell line, while in normal cells the mutants showing no or fewer cytopathic alteration were positive mutants. However, wild type influenza virus showed cytopathic alteration in both the TEVp stable cell line and normal cell line.

### 2) Purification of PROTAC influenza virus

a. When the stable cell line for rescue of mutant PROTAC influenza virus in step 1) became completely cytopathic, or about 4 days after the transfection, the cell supernatant was collected and centrifuged at 5000 g for 10 min. A massive amplification was performed with a new stable cell line, and when the cells became completely cytopathic or about 4 days after the amplification, the cell supernatant was collected and passed through a 0.45 µm filter membrane.
b. The influenza virus was purified using a method of sucrose gradient density centrifugation. The specific steps were as follows: centrifuging the viral solution in 1) in a 50 mL centrifuge tube (specific for a high speed) at 10⁵ g for 2 h, and resuspending the precipitate with 1 mL PBS.
c. Sucrose was dissolved with NTE Buffer (100 mM NaCl, 10 mM Tris-Cl, pH 7.4, 1 mM EDTA) to formulate a 20% sucrose solution, which was passed through a 0.45 µm filter membrane.
d. Sucrose from step 3) was added into a 50 mL or 15 mL centrifuge tube, and the PBS resuspension from 2) was dropped into the sucrose solution, and centrifuged at 11×10⁴ g for 2 h.
e. The precipitate is added with about 15 mL NTE buffer, and centrifugation is continued at 11 ×10⁴ g for 2 h.
f. The precipitate from step 5) was resuspended with PBS.

### Example 5 Investigation on safety of proteolysis-targeting influenza viruses at a cell level

The inventors confirmed the safety of PROTAC viruses by investigating the TEVp protein dependence of the prepared mutant PROTAC influenza viruses M1-TEVcs+PROTAC-1, M1-TEVcs+PROTAC-2, M1-TEVcs+PROTAC-1+ PROTAC-2, and M1-TEVcs+PROTAC-2+PROTAC-1, and conducted a long-term passaging culture to investigate the stability of the cleavable proteolysis-targeting molecules in the mutant viruses.

Specific experiment 1: the prepared mutant PROTAC influenza virus was used to infect MDCK-TEVp cells and normal MDCK cells at a ratio of MOI = 0.01, and the supernatant was taken after 3-4 days to measure the titers of the virus. The virus titer of PROTAC virus in MDCK-TEVp cells was set as 100%, and a relative virus titer of PROTAC virus of MDCK-TEVp cells versus normal MDCK cells was determined, so that the difference of replication capacity of the virus in the two types of cells can be known.

Specific experiment 2: freshly prepared mutant PROTAC influenza virus was inoculated in a new medium at MOI = 0.01 and infected a stable cell line, the new medium contained 1% FBS and 2 µg/mL of TPCK-trypsin, and a normal cell line was used as a control. When the TEVp stable cell line became cytopathic completely, the supernatant was taken and passed through a 0.45 µm filter membrane, and then inoculated into a new medium at a ratio of MOI = 0.01 and infected a stable cell line. Similarly, a normal cell line was used as a control. This procedure was repeated for a long-term virus passage. The introduced proteolysis-targeting molecule was tested for mutagenesis by gene sequencing.

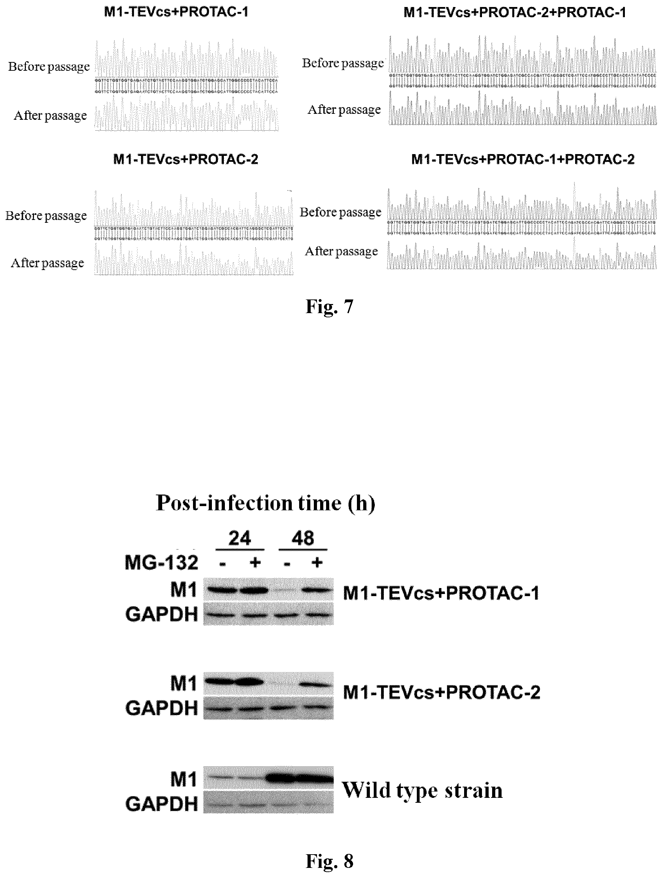

As can be seen from Figure 6, PROTAC virus can replicate and proliferate in MDCK-TEVp cells, while its replication capacity was significantly reduced or even disappeared in normal MDCK cells, i.e., it has a dependence on TEVp, indicating that the virus is safe. As can be seen from Figure 7, the proteolysis-targeting molecule introduced into the mutant PROTAC virus strain was not mutated after a long-period passage. This indicates that the cleavable proteolysis-targeting molecule introduced into an influenza virus gene is stably present, and in turn that it is genetically stable.

### Example 6 Investigation of whether diminishment of replication capacity of proteolysis-targeting influenza viruses is regulated by proteasome in cells

Using M1-TEVcs+PROTAC-1 and M1-TEVcs+PROTAC-2 as representative strains, the inventors investigated whether the diminishment of replication capacity of the designed PROTAC influenza virus in normal cells was mediated by the intracellular ubiquitin-proteasome system.

Specific experiment 1: the prepared mutant PROTAC influenza virus or wild type virus was used to infect normal MDCK cells (MOI=0.1), with a medium supplemented with 100 nM proteasome inhibitor MG-132 or DMSO diluted at the same ratio as a control. Cell samples were collected at 24 h and 48 h after the infection, respectively, and viral M1 protein levels were detected by western blot.

Specific experiment 2: the prepared mutant PROTAC influenza virus or wild type virus was used to infect MDCK-TEVp cells and normal MDCK cells (MOI = 0.01), with a medium supplemented with different concentrations (0, 50, or 100 nM) of the proteasome inhibitor MG-132 or DMSO diluted at the same ratio as a control. At 48 h after the infection, the cells were fixed with 4% PFA, and the viral NP protein levels were detected by immunofluorescence assay.

As can be seen from Figure 8, wild type virus can replicate in a large quantity and produce a large amount of viral proteins after infecting MDCK cells. In contrast, after the viral protein M1 of PROTAC virus was modified with the proteolysis-targeting molecule TEVcs+PROTAC-1 or TEVcs+PROTAC-2, the viral M1 protein was degraded after 48 hours post-infection. The proteasome-mediated degradation of viral protein M1 was inhibited after the addition of the proteasome inhibitor MG-132 into the medium. The results of this experiment indicate that the proteolysis-targeting molecule introduced by the inventors can mediate the degradation of viral protein, and the reduction of replication capacity of PROTAC virus in normal cells is caused by proteasome-mediated degradation of viral protein, which are in accordance with the principles of the present invention.

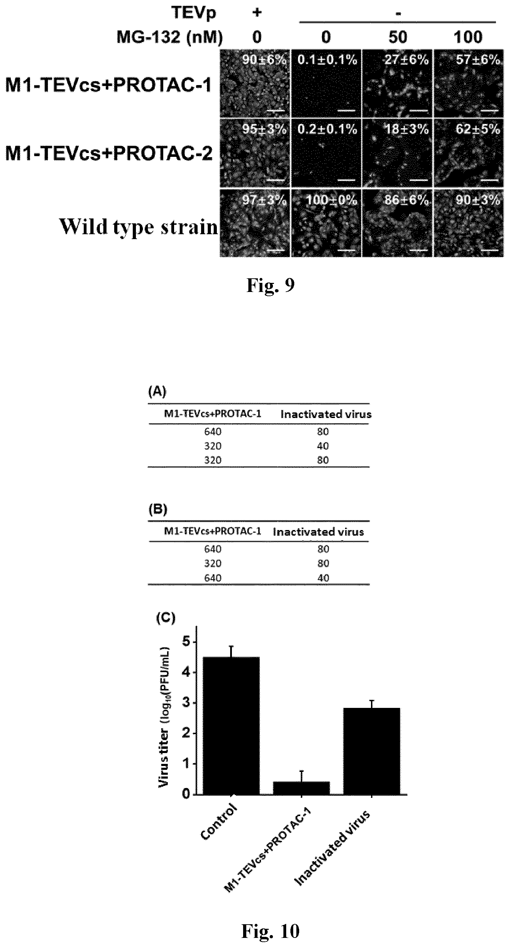

As can be seen from the results of immunofluorescence experiments in Figure 9, PROTAC viruses can replicate in a large quantity and a large amount of viral proteins were synthesized after infecting MDCK-TEVp cells. However, PROTAC viruses could not replicate in a large quantity after infecting normal MDCK cells, so less signals of viral protein NP were detected, while after the proteasome system in the cells was inhibited, the signals of viral protein NP increased, indicating that after the proteasome system was inhibited, the replication capacity of the viruses was enhanced. These results are consistent with those in Figure 8, further demonstrating that the introduction of proteolysis-targeting molecule will mediate the degradation of viral proteins by the proteasome in cells, which in turn inhibit the replication capacity of the virus, and after the proteasome system in cell is inhibited, the replication capacity of the virus will be restored.

The above results demonstrate that the reduction or defect in the replication capacity of PROTAC virus is mediated by the ubiquitin-proteasome system in cells, and this is in accordance with the inventors' design expectations for PROTAC virus.

### Example 7 Investigation of immunogenicity and protection of proteolysis-targeting influenza virus (PROTAC influenza virus) at an animal level

The immunogenicity and efficacy of PROTAC virus at an animal level was evaluated in this study using ferret (provided by Cay Ferret Farm, Wuxi, Jiangsu, China). With an inactivated influenza vaccine (IIV) as a control (the inactivated influenza virus vaccine was prepared by the inventors using homologous influenza virus particles according to the method provided by the Chinese Pharmacopoeia), M1-TEVcs+PROTAC-1 was selected as a representative of PROTAC virus for the evaluation of immunogenicity and protection of PROTAC virus.

Specific experiment:
1) Nine female ferrets aged 4-6 months were divided into three groups with three animals in each group.
2) Virus vaccination: the first group was vaccinated intranasally with PBS, the second group was vaccinated intranasally with 10⁵ PFU M1-TEVcs-PROTAC-1, and the third group was vaccinated with the same dose of inactivated virus.
3) Three weeks after the vaccination, serum was collected from each group for hemagglutination inhibition (HI) test and neutralizing (NT) antibody detection.
4) Three weeks after the vaccination, each group of animals were vaccinated intranasally with 10⁷ PFU of wild type virus WSN.
5) Three days after the vaccination with wild type virus, lung tissue was taken and the virus content therein was measured by plaque assay.

The results are shown in Figure 10. PROTAC virus could induce high levels of hemagglutination-inhibiting antibody titer (A) and neutralizing antibody titer (B) in the animals. The levels of hemagglutination-inhibiting and neutralizing antibodies induced by PROTAC virus were significantly higher than those induced by the inactivated vaccine. PROTAC virus vaccination significantly reduced the titer of wild type virus (C) in the lung tissues of the animals, and PROTAC virus vaccine provided a significantly better protection than that provided by the inactivated vaccine.

The above description is only some embodiments of the present invention. For a person of ordinary skill in the art, several variations and modifications can be made without departing from the inventive concept of the present invention, and all these fall within the protection scope of the present invention.

### References

[1] Kathleen M. Sakamoto et al. Protacs: Chimeric molecules that target proteins to the Skp1-Cullin-F box complex for ubiquitination and degradation. PNAS (2001).
[2] Ashley R. Schneekloth et al. Targeted intracellular protein degradation induced by a small molecule: En route to chemical proteomics. Bioorganic & Medicinal Chemistry Letters (2008).
[3] Dennis L. Buckley et al. Small - Molecule Inhibitors of the Interaction between the E3 Ligase VHL and HIF1α. Angewandte Chemie International Edition (2012).
[4] Daniel P Bondeson et al. Catalytic in vivo protein knockdown by small-molecule PROTACs. Nature Chemical Biology (2015).
[5] Georg E. Winter et al. Phthalimide conjugation as a strategy for in vivo target protein degradation. Science (2015).
[6] Michael Zengerle et al. Selective Small Molecule Induced Degradation of the BET Bromodomain Protein BRD4. ACS Chemical Biology (2015).
[7] Dennis L. Buckley et al. HaloPROTACS: Use of Small Molecule PROTACs to Induce Degradation of HaloTag Fusion Proteins. ACS Chemical Biology (2015).
[8] Jing Lu et al. Hijacking the E3 Ubiquitin Ligase Cereblon to Efficiently Target BRD4. Chemical Biology (2015).
[9] Yonghui Sun et al. PROTAC-induced BTK degradation as a novel therapy for mutated BTK C481S induced ibrutinib-resistant B-cell malignancies. Cell Research (2018).
[10] Yonghui Sun et al. Degradation of Bruton's tyrosine kinase mutants by PROTACs for potential treatment of ibrutinib-resistant non-Hodgkin lymphomas. Leukemia (2019).
[11] Richard R. Furman et al. Ibrutinib resistance in chronic lymphocytic leukemia. NEJM (2014).
[12] Mariell Pettersson et al. PROteolysis TArgeting Chimeras (PROTACs)-Past, present and future. Drug Discovery Today: Technologies (2019)).

## Claims

1. A proteolysis-targeting virus, comprising one or more proteolysis-targeting molecules at one or more different sites of viral protein thereof that can be recognized by the ubiquitin-proteasome system, and the viral protein is linked to the proteolysis-targeting molecule by one or more linkers, wherein the linker can be selectively cleaved.

2. The proteolysis-targeting virus according to claim 1, wherein the site is the C-terminus and/or N-terminus of viral protein;
preferably, the proteolysis-targeting molecule is any amino acid sequence selected from those as shown in SEQ ID NO: 1-110;
preferably, the linker is a molecule that can be selectively cleaved; more preferably, the linker is an amino acid sequence that can be selectively cleaved;
further preferably, the linker is selected from the group consisting of molecules that are selectively cleaved by tobacco etch virus protease, cleavable molecules that are sensitive to thrombin, molecules that are cleavable by coagulation factor Xa, molecules that are cleavable by enterokinase, molecules that are cleavable by 3C protease, molecules or sequences that are cleavable by SUMO protease, molecules that are cleavable by bacterial gelatinase, preferably such as GPLGV, and self-cleavable linker;
still further preferably, the self-cleavable linker is a 2A short peptide, and preferably the 2A short peptide is selected from the group consisting of P2A of porcine teschovirus-1, E2A of equine rhinitis A virus, F2A of foot and mouth disease virus, and self-cleavable T2A;
preferably, the linker selectively cleaved by tobacco etch virus protease is a sequences as shown in the following general formula I:
E-Xₐₐ-Xₐₐ-Y-Xₐₐ-Q-(G/S/M) I;
more preferably, the linker is any amino acid sequence selected from those as shown in SEQ ID NO: 111-137;
preferably, a flexible connector is also comprised between the proteolysis-targeting molecule and the linker;
more preferably, the proteolysis-targeting molecule, linker and flexible connector are linked in the following mode:
flexible connector-linker-flexible connector-proteolysis-targeting molecule;
further preferably, the flexible connector-linker-flexible connector-proteolysis-targeting molecule is any amino acid sequence selected from those as shown in SEQ ID NO: 138-149, 167 and 168.

3. The proteolysis-targeting virus according to claim 1 or 2, wherein the virus is selected from the group consisting of influenza virus, HIV, hand-foot-mouth virus, coxsackievirus, hepatitis C virus HCV, hepatitis B virus HBV, hepatitis A virus, hepatitis D virus, hepatitis E virus, EB virus, human papilloma virus HPV, herpes simplex virus HSV, cytomegalovirus, varicella-zoster virus, vesicular stomatitis virus, respiratory syncytial virus RSV, dengue virus, Ebola virus, Marburg virus, Zika virus, SARS, Middle East respiratory syndrome virus, rotavirus, rabies virus, measles virus, adenovirus, poliovirus, echovirus, encephalitis B virus, forest encephalitis virus, hantavirus, novel enterovirus, rubella virus, mumps virus, parainfluenza virus, blue ear virus, swine fever virus, foot-and-mouth disease virus, microvirus, prion virus, smallpox virus, tobacco mosaic virus, adeno-associated virus, phage, herpes virus, West Nile virus, Norovirus, human boca virus, coronavirus and novel coronavirus SARS-CoV-2; and more preferably, the virus is influenza virus or novel coronavirus SARS-CoV-2;
preferably, the virus is a modified virus.

4. The proteolysis-targeting virus according to any of claims 1-3, which is a proteolysis-targeting influenza virus comprising one or more proteolysis-targeting molecules at one or more different sites of viral protein thereof that can be recognized by the ubiquitin-proteasome system, and the viral protein is linked to the proteolysis-targeting molecule by a linker, wherein the linker is E-Xₐₐ-Xₐₐ-Y-Xₐₐ-Q-(G/S/M), which can be specifically recognized and cleaved by tobacco etch virus protease;
preferably, the virus is H1N1, H5N1, H7N9, H3N2 or influenza B virus; more preferably, one or more of PA, PB1, PB2, NP, HA, NA, M1, M2, NS1, and NEP proteins of influenza virus comprise a proteolysis-targeting molecule and linker;
further preferably, both of PA and PB2 of the influenza virus comprise one or more proteolysis-targeting molecules and linkers;
both of PA protein and PB1 protein of the influenza virus comprise one or more proteolysis-targeting molecules and linkers;
both of PB2 protein and PB1 protein of the influenza virus comprise one or more proteolysis-targeting molecules and linkers;
all of PA protein, PB2 protein, and PB1 protein of the influenza virus comprise one or more proteolysis-targeting molecules and linkers;
all of PA protein, PB2 protein, PB1 protein, and M1 protein of the influenza virus comprise one or more proteolysis-targeting molecules and linkers;
all of PA protein, PB2 protein, PB1 protein, M1 protein, and NP protein of the influenza virus comprise one or more proteolysis-targeting molecules and linkers;
all of PB2 protein, PB1 protein, and M1 protein of the influenza virus comprise one or more proteolysis-targeting molecules and linkers;
both of PB1 protein and M1 protein of the influenza virus comprise one or more proteolysis-targeting molecules and linkers;
both of PB2 protein and M1 protein of the influenza virus comprise one or more proteolysis-targeting molecules and linkers;
all of PB2 protein, PB1 protein, M1 protein, and NS1 protein of the influenza virus comprise one or more proteolysis-targeting molecules and linkers;
all of PB2 protein, PB1 protein, M1 protein, and NEP protein of the influenza virus comprise one or more proteolysis-targeting molecules and linkers; or
both of NS1 protein and NEP protein of the influenza virus comprises one or more proteolysis-targeting molecules and linkers;
preferably, the proteolysis-targeting virus is a proteolysis-targeting coronavirus, comprising one or more proteolysis-targeting molecules at one or more different sites of viral protein thereof that can be recognized by the ubiquitin-proteasome system, and the viral protein is linked to the proteolysis-targeting molecule by a linker, wherein the linker is E-Xₐₐ-Xₐₐ-Y-Xₐₐ-Q-(G/S/M), which can be specifically recognized and cleaved by tobacco etch virus protease;
preferably, the virus is novel coronavirus SARS-CoV-2;
more preferably, one or more of spike protein, envelope glycoprotein, membrane glycoprotein, nucleocapsid protein, non-structural protein 1, non-structural protein 2, non-structural protein 3, non-structural protein 4, non-structural protein 5, non-structural protein 6, non-structural protein 7, non-structural protein 8, non-structural protein 9, non-structural protein 10, non-structural protein 11, non-structural protein 12, non-structural protein 13, non-structural protein 14, non-structural protein 15, non-structural protein 16, 3a protein, 3b protein, 6 protein, 7a protein, 7b protein, 8a protein, 8b protein, 9b protein, 3C-like proteinase, leader protein, 2'-O-ribose methyltransferase, endonuclease, 3'- to 5'- exonuclease, helicase, RNA-dependent RNA polymerase, orf1a polyprotein, ORF10 protein, ORF8 protein, ORF7a protein, ORF6 protein, and ORF3a protein of the coronavirus comprise one or more proteolysis-targeting molecules and linkers;
preferably, the proteolysis-targeting virus is a proteolysis-targeting HIV virus, comprising one or more proteolysis-targeting molecules at one or more different sites of viral protein thereof that can be recognized by the ubiquitin-proteasome system, and the viral protein is linked to the proteolysis-targeting molecule by a linker, wherein the linker is E-Xₐₐ-Xₐₐ-Y-Xₐₐ-Q-(G/S/M), which can be specifically recognized and cleaved by tobacco etch virus protease;
preferably, the virus is an HIV virus;
still preferably, one or more of Gag polyprotein, pol polyprotein, gp160, HIV trans-activator of transcription, regulator of expression of virion protein, viral negative factor, lentiviral protein R, viral infectivity factor, viral protein U, matrix protein, capsid protein, spacer peptide 1, nucleocapsid protein, spacer peptide 2, P6, reverse transcriptase, ribonuclease H (Rnase H), integrase, HIV protease, gp120, and gp41 protein comprise one or more proteolysis-targeting molecules and linkers .

5. A nucleic acid molecule encoding the proteolysis-targeting virus according to any one of claims 1-4.

6. A nucleic acid vector expressing the proteolysis-targeting virus according to any one of claims 1-4.

7. A method for preparing the proteolysis-targeting virus according to any one of claims 1-4, comprising the steps of:
**1**) construction of cell line: constructing a cell line that can stably express a protease capable of selective cleaving the linker of the proteolysis-targeting virus;
preferably, the cell line is a mammalian cell line;
more preferably, the cell line is selected from CHO cells, Vero cells, MDCK.2 cells, HEK293T cells, MDCK cells, A549 cells, BHK cells, BHK-21/BRS cells, Sp2/0 cells, HEK293 cells, 293F cells , HeLa cells, TZM-bl cells, Sup-T1 cells, MRC-5 cells and VMK cells, LLC-MK2 cells, HCT-8 cells, Huh-7 cells, and Caco2 cells;
further preferably, the cell line is selected from HEK293T cell line and MDCK cell line;
preferably, the cell line is optionally a ubiquitin-proteasome system-deficient cell line; more preferably, the cell line is a cell line with E3 ligase knockout or knockdown;
**2)** site selection: determining a viral protein and site into which the proteolysis-targeting molecule and linker are introduced through statistical analysis on the expression distribution of the ubiquitin-proteasome system in a host, and bioinformatic and protein structural prediction of the virus;
**3)** gene mutation: introducing a nucleotide sequence encoding the proteolysis-targeting molecule and linker into the selected site of encoding gene of determined viral protein, using a genetic engineering method;
**4)** construction of expression vector: operably linking the encoding nucleotide sequence of genetically mutated viral protein obtained in step 3) to a vector to obtain an expression vector;
preferably, the expression vector is a plasmid;
**5)** cotransfecting the expression vector in step 4) and other expression vector for rescue of influenza virus into the cell line constructed in step 1) capable of selectively cleaving the linker of the proteolysis-targeting virus using reverse genetic technology, to obtain the proteolysis-targeting virus;
optionally, **6)** replicating the proteolysis-targeting virus in the cell line obtained in step 1) to produce the proteolysis-targeting virus;
preferably, a proteasome inhibitor is added during the preparation of the virus; more preferably, the proteasome inhibitor is MG132, MG-341 or lactacystin;
or
comprising the steps of:
(i) site selection: determining a viral protein and site into which the proteolysis-targeting molecule and linker are introduced through statistical analysis on the expression distribution of the ubiquitin-proteasome system in a host, and bioinformatic and protein structural prediction of the virus;
(ii) gene mutation: introducing a nucleotide sequence encoding the proteolysis-targeting molecule and linker into the selected site of encoding gene of determined viral protein, using a genetic engineering method;
(iii) construction of expression vector for mutated sequence: operably linking the encoding nucleotide sequence of genetically mutated viral protein obtained in step ii) to a vector to obtain an expression vector;
preferably, the expression vector is a plasmid;
(iv) constructing an overexpression vector of a protease capable of selectively cleaving the linker of the proteolysis-targeting virus;
constructing a cell line that can stably express a protease capable of selectively cleaving the linker of the proteolysis-targeting virus;
(v) cotransfecting the expression vector obtained in step (iii), other expression vector required for rescue of the virus, and the expression vector obtained in step (iv) into host cells using reverse genetic technology, and culturing the host cells transfected successfully in a culture medium, to obtain the proteolysis-targeting virus.

8. The method according to claim 7, further comprising step 7):
detection: determining whether the proteolysis-targeting virus has been successfully modified by measuring the replication capacity of the proteolysis-targeting virus obtained in step 5) in the cell line obtained in step 1) and in normal host cells without being modified, wherein the proteolysis-targeting virus that replicates in the cell line obtained in step 1) and has or no reduced replication capacity in normal host cells without being modified is a successfully modified proteolysis-targeting virus;
optionally, the method further comprises step 8):
using the successfully modified proteolysis-targeting virus, repeating steps 2)-5), so that the proteolysis-targeting molecule and linker are introduced into multiple viral proteins of the proteolysis-targeting virus, or multiple proteolysis-targeting molecules and linkers are introduced into any viral protein of the proteolysis-targeting virus;
optionally, determining whether the proteolysis-targeting virus has been successfully modified by measuring the replication capacity of obtained proteolysis-targeting virus in the cell line obtained in step 1) and in normal host cells without being modified, wherein the proteolysis-targeting virus that replicates in the cell line obtained in step 1) and has reduced or no replication capacity in normal host cells without being modified is a successfully modified proteolysis-targeting virus.

9. The method according to claim 7 or 8, wherein the proteolysis-targeting virus is a proteolysis-targeting influenza virus, comprising the steps of:
1) construction of cell line: stably transducing the tobacco etch virus protease TEVp into a mammalian cell line, and constructing a cell line that can stably express the tobacco etch virus protease TEVp;
preferably, the mammalian cell line is HEK293T cell line or MDCK cell line;
2) site selection: determining the gene fragment and site into which the proteolysis-targeting molecule and linker are introduced, through statistical analysis on the expression distribution of the ubiquitin-proteasome system in a host, and bioinformatic and protein structural prediction of influenza virus, predicting and analyzing the protein structure of influenza virus into which a sequence of the proteolysis-targeting molecule and linker that is cleaved by tobacco etch virus protease are introduced;
preferably, one or more insertion sites are selected from the gene fragments encoding different proteins of influenza virus;
3) gene mutation: introducing a nucleotide sequence encoding the proteolysis-targeting molecule and linker into the selected site of encoding gene of determined influenza viral protein, using a genetic engineering method;
4) construction of plasmid: operably linking the encoding nucleotide sequence of genetically mutated viral protein obtained in step 3) to a plasmid to obtain an encoding plasmid;
5) cotransfecting the plasmid in step 4) and other plasmid for rescue of influenza virus in the cell line that stably expresses TEVp obtained in step 1) using reverse genetic technology, to obtain the proteolysis-targeting influenza virus;
optionally, 6) producing the proteolysis-targeting influenza virus in a stable cell line that stably expresses TEVp;
preferably, the method further comprises step 7):
detection: determining whether the proteolysis-targeting influenza virus has been successfully modified by determining the dependence of proteolysis-targeting influenza virus obtained in step 5) on TEVp and the dependence of inactivation of its packaged product on the proteasome pathway;
optionally, the method further comprises step 8):
using the successfully modified proteolysis-targeting influenza virus vector, repeating steps 2)-5), so that the proteolysis-targeting molecule and linker are introduced into each of multiple viral proteins of the proteolysis-targeting influenza virus, or multiple proteolysis-targeting molecules and linkers are introduced into any viral protein of the proteolysis-targeting influenza virus;
preferably, determining whether the proteolysis-targeting virus has been successfully constructed by determining the dependence of obtained proteolysis-targeting influenza virus on TEVp and the dependence of inactivation of its packaged product on the proteasome pathway, and retaining the proteolysis-targeting virus that still maintains the dependence on TEVp after a long-term passage as a successfully modified candidate;
optionally, the method further comprises:
step 9): selecting the successfully modified candidate and purifying the product;
step 10): performing a safety or immunogenicity detection on the proteolysis-targeting influenza virus in step 9), and compared with the wild type virus, the safer influenza virus is a successfully modified influenza virus.

10. A method for preparing an attenuated live virus, replication-incompetent live virus, replication-controllable live virus, and for preparing a relevant vaccine and medicament for preventing and treating viral infections, comprising a step of using the proteolysistargeting virus of any of claims 1-4, or a step of preparing a proteolysis-targeting virus using the method of any of claims 7-9.

11. A vaccine or pharmaceutical composition, comprising the proteolysis-targeting virus according to any one of claims 1-4;
preferably, the vaccine is an attenuated live vaccine, replication-incompetent live vaccine, or replication-controllable live vaccine.

12. A system for preparing the proteolysis-targeting virus according to any one of claims 1-4, comprising:
a cell line that stably expresses a protease capable of selectively cleaving the linker of the proteolysis-targeting virus;
preferably, the cell line is a cell line stably expressing the tobacco etch virus protease TEVp;
more preferably, the cell line is HEK293T cell line or MDCK cell line stably expressing the tobacco etch virus protease TEVp;
further preferably, the cell line is optionally a ubiquitin-proteasome system-deficient cell line; preferably, the cell line is a cell line with E3 ligase knockout or knockdown;
still further preferably, the system further comprises a nucleic acid vector expressing the proteolysis-targeting virus according to any one of claims 1-4.
